# EUROPEAN PATENT SPECIFICATION

(11) **EP 2 207 779 B1**
(45) Date of publication and mention of the grant of the patent: **09.04.2014**
(21) Application number: 08850132.5
(22) Date of filing: 14.11.2008
(51) Int. Cl.: C07D 473/18, A61K 31/70

(54) **METHOD OF SYNTHESIS OF MORPHOLINO OLIGOMERS**
VERFAHREN ZUR SYNTHESE VON MORPHOLINO-OLIGOMEREN
PROCÉDÉ DE SYNTHÈSE D'OLIGOMÈRES MORPHOLINO

(30) Priority: 15.11.2007 US 988200 P; 15.11.2007 US 988192 P
(43) Date of publication of application: 21.07.2010
(73) Proprietor: Sarepta Therapeutics, Inc., Bothell, Washington 98021 (US)
(72) Inventor: FOX, Christina, Mary Josephine, Corvallis OR 97333 (US); REEVES, Matthew, Dale, Albany OR 97322 (US); WELLER, Dwight, D., Corvallis OR 97330 (US); LI, Yongfu, Corvallis Oregon 97330 (US)
(74) Representative: Schiweck, Wolfram
(86) International application number: PCT/US2008/012804
(87) International publication number: WO 2009/064471

(56) References cited:
- WO-A-2008/008113
- WO-A-2008/036127
- US-A- 5 185 444
- US-A1- 2007 135 333
- SUMMERTON ET AL: "Morpholino antisense oligomers: design, preparation, and properties" SENSE & NUCLEIC ACID DRUG DEVELOPMENT, MARY ANN LIEBERT, INC., NEW YORK, US, vol. 7, 1 January 1997 (1997-01-01), pages 187-195, XP002136176 ISSN: 1087-2906 cited in the application
- CASTRO M J L ET AL: "Gemini surfactants from alkyl glucosides" TETRAHEDRON LETTERS 19970609 GB, vol. 38, no. 23, 9 June 1997 (1997-06-09), pages 3995-3998, XP002513601 ISSN: 0040-4039
- ABRAMOVA T V ET AL: "Synthesis of morpholine nucleoside triphosphates" TETRAHEDRON LETTERS, ELSEVIER, AMSTERDAM, vol. 45, no. 22, 24 May 2004 (2004-05-24), pages 4361-4364, XP004506361 ISSN: 0040-4039
- ABRAMOVA TATIANA V ET AL: "New oligonucleotide analogues based on morpholine subunits joined by oxalyl diamide tether." BIOORGANIC CHEMISTRY JUN 2007, vol. 35, no. 3, June 2007 (2007-06), pages 258-275, XP002513602 ISSN: 0045-2068
- March: "Advanced Organic Chemistry", John Willey & SONS

### SEQUENCE LISTING

<110> AVI BIOPHARMA, INC.
<120> Method of synthesis of Morpholino oligomers
<130> P43921
<140> EP 08850132.5
   <141> 2008-11-14
<150> US 60/988,192
   <151> 2007-11-15
<150> US 60/988,200
   <151> 2007-11-15
<160> 4
<170> PatentIn version 3.5
<210> 1
   <211> 20
   <212> DNA
   <213> Artificial sequence
<220>
   <223> Synthetic oligomer
<400> 1
   acgttgaggg gcatcgtcgc 20
<210> 2
   <211> 20
   <212> DNA
   <213> Artificial Sequence
<220>
   <223> synthetic oligomer
<400> 2
   ctgggatgag agccatcact 20
<210> 3
   <211> 22
   <212> DNA
   <213> Artificial sequence
<220>
   <223> synthetic oligomer
<400> 3
   cttagtcatc gagatcttcg tg 22
<210> 4
   <211> 19
   <212> DNA
   <213> Artificial Sequence
<220>
   <223> Synthetic oligomer
<400> 4
   gtgctcatgg tgcacggtc 19

## Description

### Field of the Invention

The invention relates to methods of synthesizing phosphorodiamidate-linked morpholino oligomers by coupling of morpholino subunit monomers, and in particular to improved procedures for deprotection of the protected morpholino ring nitrogen at each coupling step, and to the use of guanine morpholino (MoG) subunits with protection at both the N2 and 06/N1 groups of the guanine base. Morpholino oligomers synthesized using these modifications are obtained in higher purity and yield compared to those synthesized using monoprotected guanine subunits and/or conventional ring nitrogen deprotection procedures.

### References

Albert, A., Physical Methods in Heterocyclic Chemistrys, Vol. I, A.R. Katritzky, Ed., Academic Press, pp 44 (1963).
Fisher, A., Galloway, W.J., and Vaughan, J., J. Chem. Soc. 3591 (1964).
Garrison, A. W. and Boozer, C.E., J. Am. chem. Soc. 90(13):3486-3494 (1968).
Gough et al. (1979) Nucleic Acids, Research 7:1955-1964.
Hata et al. (1983) Tetrahedron Lett. 24:2775-2778.
Jones et al. (1982A) Tetrahedron Lett. 23:2253-2256.
Jones et al. (1982B) Tetrahedron Lett. 23:2257-2260.
Mitsunobu, O. (1981) Synthesis 1:1-28.
Ravikumar, V. et al., U.S. Patent No. 5,510,476.
- Reese et al. (1981) Tetrahedron Lett: 22:4755-4758.
Reese et al. (1984) J.Chem.Soc., Perkin Trans. I 1263-1270.
Rogne, O., J. Chem. Soc. 727 (1970).
Summerton, J.E. and Weller, D.D. (1993) U.S, Patent No. 5, 185,444.
Summerton, J.E. and Weller, D.D., Antisense Nucl. Acid Drug Dev. 7(3):187-195 (1997).

### Background

Phosphorodiamidate-linked morpholino oligomers, or PMO, arc nucleic acid analogs which bind tightly and sequence-specifically to complementary RNA and are useful in modulating protein synthesis and thus gene expression. These oligomers are composed of base-pairing recognition moieties (heterocyclic bases) supported by a morpholino backbone system. Morpholino subunits for use in synthesizing such oligomers can be prepared easily from the corresponding ribonucleosides, which are readily available and inexpensive precursors (see e.g. Summerton and Weller, 1993, 1997).

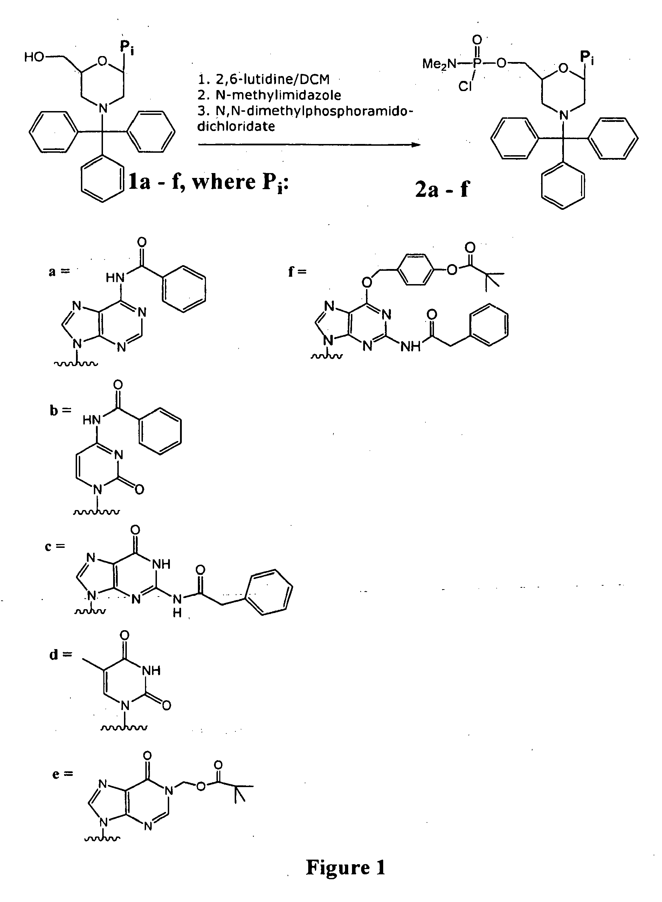

During such synthesis, as in conventional oligonucleotide synthesis, the functional groups on the heterocyclic bases are typically masked to prevent interference in the synthetic transformations. For example, activation of the N-tritylated morpholino monomer (**1a-f**; Figure 1) entails reaction of the 5'-hydroxyl with a suitable phosphoramido dichloridate to form the activated subunit **2a-f**. At large scale (50-100 Gallon reactor), the crude activated subunit is generally contaminated with a high level of by-products. Following chromatographic purification, the activated subunit is isolated in about 50% yield for A, C, 1, T, U and their protected forms, but only in about 5% yield for the activated singly protected G subunit, which is believed to be due to the presence of the unprotected 06 oxygen.

The 06-unprotected guanine subunit also gives rise to side reactions at the oligomer stage. For example, the 06 oxygen can react with activated subunit during coupling steps, to form 06-phosphorylated or derivative species, and during final cleavage of the base protecting groups with ammonia, ammonia can react at C6 to displace these species, giving a diaminopurine derivative. Such impurities are difficult to remove by chromatography, and cause a large loss in yield.

Various protection schemes have been proposed in the art to reduce side reactions of unprotected guanine 06 positions in conventional oligonucleotide synthesis (see e.g. Gough *et al.* 1979; Reese *et al.* 1981, 1984; Jones *et al.* 1982A, 1982B). However, these protocols were largely unsuccessful when applied to PMO synthesis. Accordingly, improved methods are sought to increase yield and purity in PMO synthesis, particularly in the use of G morpholino subunits.

The morpholino nitrogen of a morpholino subunit is also protected prior to use, typically with a trityl or substituted trityl species. During oligomer synthesis, this group must be removed during each cycle to allow incorporation of the next subunit. Failure to completely remove the protecting group leads to N-1 deletion sequences that contaminate the desired oligomer product.

Trityl groups are conventionally removed with acid, and deprotecting reagents used for PMO synthesis have traditionally been carboxylic acids (Summerton *et al.* 1993, 1997). However, phosphorodiamidate groups are also sensitive to acid, and carboxylic acids useful for detritylation are also capable of promoting hydrolysis of phosphorodiamidate linkages to amidate species, as shown in Fig. 1, with the possibility of more extensive backbone degradation. For example, cyanoacetic acid in 20% acetonitrile/DCM is an effective deprotecting reagent, but it is found to cause substantial (5-10%) hydrolysis of phosphorodiamidate linkages in the PMO product.

Carboxylic acids must also be completely removed from the synthesis support resin prior to the coupling reaction; otherwise, by-products are formed that consist of truncated oligomers containing a 3'-acylated species.

US 5185444 (A) discloses a polymer composition comprising: morpholino subunit structures linked together by uncharged, chiral linkages, one-three atoms in length. These chiral linkages join the morpholino nitrogen of one subunit to the 5' exocyclic carbon of an adjacent subunit. Each subunit contains a purine or pyrimidine base-pairing moiety effective to bind by hydrogen bending to a specific base or base-pair in a target polynucleotide.

US2007135333 (A1) relates to an antibacterial antisense conjugate and a method of using the same for treating a bacterial infection in a mammalian host. The conjugate includes a substantially uncharged antisense oligonucleotide conjugated to a carrier peptide that significantly enhances the antibacterial activity of the oligonucleotide. The antisense oligonucleotide contains 10-20 nucleotide bases and has a targeting nucleic acid sequence complementary to a target sequence containing or within 10 bases, in a downstream direction, of the translational start codon of a bacterial mRNA that encodes a bacterial protein essential for bacterial replication, where the compound binds to a target mRNA with a Tm of between 50 ° to 60 °C. The carrier peptide is an arginine-rich peptide containing between 6 and 12 amino acids.

WO2008/008113 (A1) discloses a method for enhancing, by at least 10 fold, the antibacterial activity of an antisense oligonucleotide composed of morpholino subunits linked by phosphorus-containing intersubunit linkages. The method includes one or both of: conjugating an arginine-rich carrier to a 3' or 5' end of the oligonucleotide and modifying the oligonucleotide to contain 20%-50% intersubunit linkages that are positively charged at physiological pH.

WO2008/036127 (A2) discloses morpholino oligomers that contain both uncharged and cationic intersubunit linkages. The oligomers are oligonucleotide analogs containing predetermined sequences of base-pairing moieties. The presence of the cationic intersubunit linkages in the oligomers, typically at a level of about 10-50% of total linkages, is reported to provide enhanced antisense activity relative to the corresponding uncharged oligomers. WO2008/036127 also discloses such oligomers conjugated to peptide transporter moieties, where the transporters are preferably composed of arginine subunits, or arginine dimers, alternating with neutral amino acid subunits.

For the above reasons, improved reagents are needed for morpholino nitrogen deprotection in PMO synthesis.

### Summary

In one aspect, the invention provides a morpholino compound comprising the structure **I:** 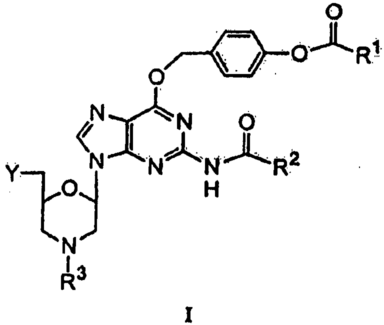 wherein
R¹ is selected from the group consisting of lower alkyl, di(lower alkyl)amino; and phenyl;
R² is selected from the group consisting of lower alkyl, monocyclic arylmethyl, and monocyclic (aryloxy)methyl;
R³ is selected from the group consisting of triarylmethyl and hydrogen; and
Y is selected from the group consisting of a protected or unprotected hydroxyl or amino group; a chlorophosphoramidate group; and a phosphorodiamidate linkage to the ring nitrogen of a further morpholino compound a phosphorodiamidate linkage to the morpholino ring nitrogen of a morpholino subunit in a morpholino oligomer or a solid support. The term lower alkyl defines an alkyl radical of one to six carbon atoms. The compound does not have the following structure: 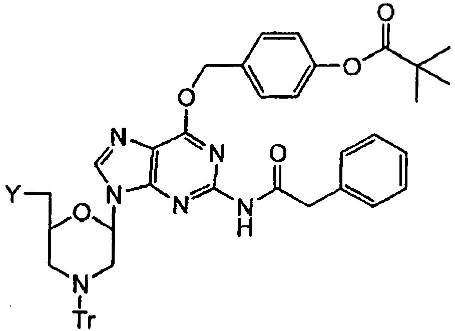
In this structure:
Y is -OH, 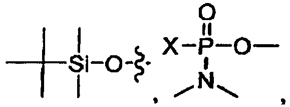 where X is Cl, the morpholino ring nitrogen of a further morpholino compound, the morpholino ring nitrogen of a morpholino subunit in a morpholino oligomer, or a solid support. In the above structure Tr is trityl.

In selected embodiments, Y is selected from the group consisting of a protected or unprotected hydroxyl group and a chlorophosphoramidate group, e.g. a chlorophosphoramidate group of the form -O-P(=O)-N(CH₃)₂Cl. When Y is a protected hydroxyl group, it is preferably a trialkylsilyl-protected hydroxyl group.

The group R³ is preferably selected from trityl (triphenylmethyl), 4-methoxytrityl, 4-methyltrityl, 4,4'-dimethyltrityl, and 4,4',4"-trimethyltrityl. The group R¹ is preferably lower alkyl, especially C₁-C₄ alkyl, and most particularly -C(CH₃)₃ (tert-butyl). The group R² is preferably selected from benzyl and -CH(CH₃)₂ (isopropyl).

In a related aspect, the invention provides an improved method of synthesizing a morpholino oligomer, the method comprising:
(a) reacting a solid-phase-supported morpholino subunit, having an unprotected ring nitrogen, with a first morpholino subunit monomer, having a triarylmethyl-protected ring nitrogen and an activated phosphoramidate group on a 5'-exocyclic carbon, thereby forming a phosphorodiamidate linkage between the 5'-exocyclic carbon and the unprotected ring nitrogen;
(b) deprotecting the protected ring nitrogen, to form an unprotected ring nitrogen;
(c) optionally reacting the product from step (b) with a further morpholino subunit monomer comprising: a triarylmethyl-protected morpholino ring nitrogen and an activated phosphoramidate group on a 5'-exocyclic carbon, thereby forming a phosphoramidate linkage between the 5'-exocyclic carbon of the further morpholino subunit monomer and the unprotected morpholino ring nitrogen of the product from step (b); and
(d) optionally repeating steps (b) and (c) one or more times.

In this method at least one of the first morpholino subunit monomer, the further morpholino subunit monomer or the solid-phase-supported morpholino subunit monomer is a doubly protected guanine morpholino compound having the structure 1: 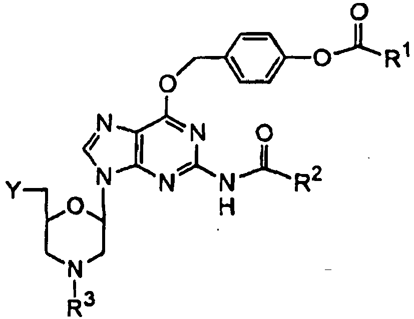
In this structure R¹ is selected from the group consisting of lower alkyl, di(lower alkyl)amino, and phenyl;
R² is selected from the group consisting of lower alkyl, monocyclic arylmethyl, and monocyclic (aryloxy)methyl;
R³ is selected from the group consisting of triarylmethyl and hydrogen; and
Y is a chlorophosphoramidate group or a solid support.

In this regard the term lower alkyl defines an alkyl radical of one to six carbon atoms.

The doubly protected guanine morpholino compound does not have the following structure: 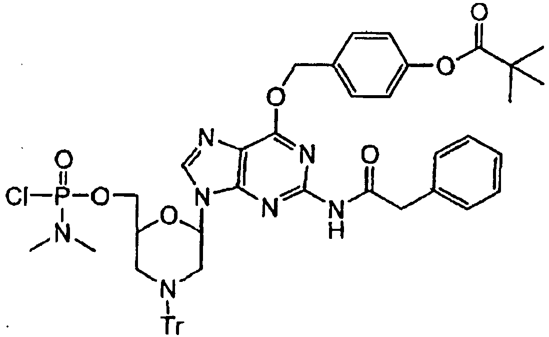 or: 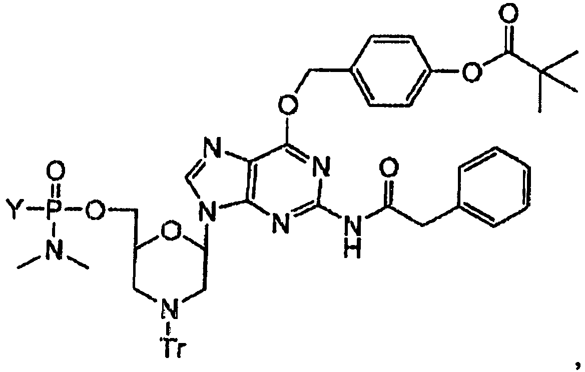
wherein Y is a solid support and Tr is trityl.

Selected embodiments of the variables represented in the above structure include those described above.

In a further aspect, the invention provides an improved method of synthesizing a morpholino oligomer, the method comprising:
(a) reacting a solid-phase-supported morpholino subunit, having an unprotected ring nitrogen, with a base-protected morpholino subunit monomer, having a triarylmethyl-protected ring nitrogen and an activated phosphoramidate group on a 5'-exocyclic carbon, thereby forming a phosphorodiamidate linkage between the 5'-exocyclic carbon and the unprotected ring nitrogen;
(b) deprotecting the protected ring nitrogen, to form an unprotected ring nitrogen; and
(c) repeating steps (a) and (b) one or more times with further base-protected morpholino subunit monomers;
wherein said deprotecting comprises exposing the triarylmethyl-protected ring nitrogen to a reagent solution comprising a heterocyclic amine salt in a trifluoroethanol-containing solvent, the salt being a salt of a heterocyclic amine, having a pKa in the range of 1-4 in its protonated form, with an acid selected from a sulfonic acid, trifluoroacetic acid, and hydrochloric acid.

The heterocyclic amine is preferably selected from the group consisting of: an electron withdrawing group-substituted pyridine, thiazole, pyridazine, pyrazole, triazole and electron withdrawing group-substituted substituted derivatives of these. Such electron withdrawing groups (EWG) include halogen, cyano, aldehyde, keto, carboxyester, and carboxamide.

Preferably, the heterocyclic amine is an electron withdrawing group-substituted pyridine, such as a chloro- or cyano-substituted pyridine. The amine salt is preferably a salt of a sulfonic acid, such as an alkylsulfonate, (fluoroalkyl)sulfonate, or p-toluenesulfonate, or a trifluoroacetate. In selected embodiments, the salt is selected from - 3-chloropyridinium methanesulfonate (CPM) and 4-cyanopyridinium trifluoroacetate (CYTFA).

The TFE-containing solvent preferably comprises dichloromethane and trifluoroethanol in volume ratio in the range of about 90:10 to 25:75, and more preferably in a volume ratio of about 80:20 DCM:TFE.

The triarylmethyl protecting group is selected from the group consisting of trityl (triphenylmethyl), 4-methoxytrityl, 4-methyltrityl, 4,4'-dimethyltrityl, and 4,4',4"-trimethyltrityl.

The modifications and improvements described herein may be combined, such that steps (a) - (c) above are carried out wherein:
(i) at least one of the base-protected morpholino subunit monomers is a doubly protected guanine morpholino compound having the structure **I** as set forth above; and
(ii) deprotecting the protected ring nitrogen comprises exposing the triarylmethyl-protected ring nitrogen to a reagent solution comprising a heterocyclic amine salt in a trifluoroethanol-containing solvent, the salt being a salt of a heterocyclic amine, having a pKa in the range of 1-4 in its protonated form, with an acid selected from a sulfonic acid, trifluoroacetic acid, and hydrochloric acid.

Typically, the synthesis further comprises cleaving the morpholino oligomer from the solid phase and deprotecting the bases, in accordance with standard procedures.

These and other objects and features of the invention will become more fully apparent when the following detailed description of the invention is read in conjunction with the accompanying drawings.

### Brief Description of the Drawings

Figure 1 illustrates the formation of an activated morpholino subunit.
Figure 2 illustrates a route of formation for a doubly protected morpholino G subunit (DPG) derivative in which the N2 position is phenylacetylated and the 06 position is protected with the 4-nitrophenethyl (NPE) group.
Figures 3 illustrates an alternate route of formation for a doubly protected morpholino G subunit (DPG) derivative in which the N2 position is phenylacetylated and the 06 position is protected with the 4-nitrophenethyl (NPE) group.
Figure 4 illustrates the formation of a DPG derivative in which the N2 position is phenylacetylated and the 06 position is protected with either the phenylsulfonylethyl (PSE) or methylsulfonylethyl (MSE) group.
Figure 5 illustrates the formation of a DPG derivative in which the N2 position is phenylacetylated and the 06 position is protected with the trimethylsilylethyl (TMSE) group.
Figure 6 illustrates the formation of a DPG derivative in which the N2 position is phenylacetylated and the 06 position is protected with a series of aryl derivatives.
Figure 7 illustrates the formation of a DPG derivative in which the N2 position is phenylacetylated and the 06 position is protected with a series of carbamoyl derivatives.
Figure 8 illustrates the formation of the DPG derivative in which the N2 position is phenylacetylated and the 06 position is protected with the 4-(pivaloyloxy)benzyloxy (POB) group.

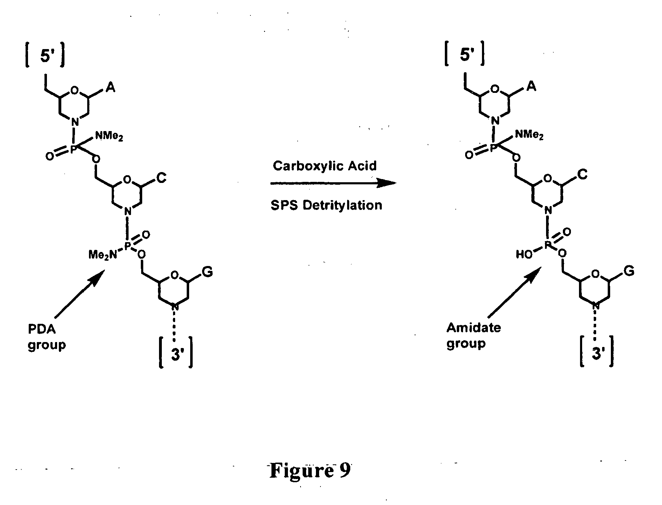

Figure 9 shows conversion of the phosphorodiamidate (PDA) linkage into the phosphoramidate (amidate) linkages, in a side reaction that can occur upon treatment of phosphorodiamidate-linked morpholino oligomers (PMO) with carboxylic acids.
Figure 10 illustrates the preparation of a disulfide anchor, for use in modification of a synthesis resin used for stepwise preparation of a morpholino oligomer, allowing facile release of the oligomer by treatment with a thiol.
Figure 11 illustrates the preparation of a triethylene glycol containing moiety ("Tail") which increases aqueous solubility of synthetic antisense oligomers.
Figure 12 illustrates, the preparation of resins useful for the solid phase synthesis of morpholino oligomers.

### Detailed Description of the Invention

### 1. Definitions

The terms below, as used herein, have the following meanings, unless indicated otherwise:
A "morpholino oligomer" refers to a polymeric molecule having a backbone which supports bases capable of hydrogen bonding to typical polynucleotides, wherein the polymer lacks a pentose sugar backbone moiety, and more specifically a ribose backbone linked by phosphodiester bonds which is typical of nucleotides and nucleosides, but instead contains a ring nitrogen with coupling through the ring nitrogen. A preferred morpholino oligomer is composed of "morpholino subunit" structures, such as shown below, which in the oligomer are preferably linked together by (thio)phosphorodiamidate linkages, joining the morpholino nitrogen of one subunit to the 5' exocyclic carbon of an adjacent subunit. Each subunit includes a purine or pyrimidine base-pairing moiety Pi which is effective to bind, by base-specific hydrogen bonding, to a base in a polynucleotide.

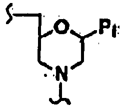

Morpholino oligomers are detailed, for example, in co-owned U.S. Patent Nos. 5,698,685, 5,217,866, 5,142,047, 5,034,506, 5,166,315, 5,185,444, 5,521,063, and 5,506,337.

A "phosphorodiamidate" group comprises phosphorus having two attached oxygen atoms and two attached nitrogen atoms, and herein may also refer to phosphorus having one attached oxygen atom and three attached nitrogen atoms. In the intersubunit linkages of the oligomers described herein, one nitrogen is typically pendant to the backbone chain, and the second nitrogen is the ring nitrogen in a morpholino ring structure, as shown in formula **II** below. Alternatively or in addition, a nitrogen may be present at the 5'-exocyclic carbon, as shown in formulas **III** and **IV** below.

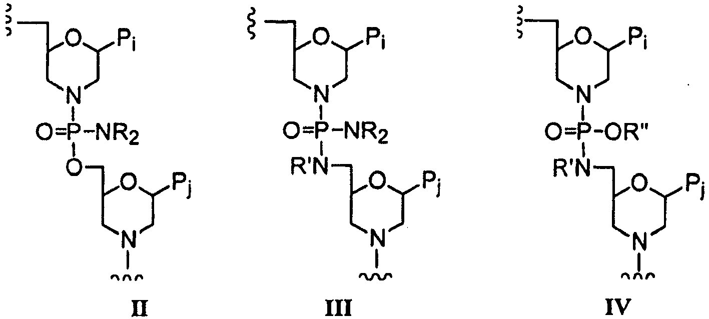

In a thiophosphorodiamidate linkage, one oxygen atom, typically an oxygen pendant to the backbone in the oligomers described herein, is replaced with sulfur.

A "solid-phase-supported morpholino subunit" can be the first or any subsequent morpholino subunit monomer incorporated into a morpholino oligomer by solid-phase stepwise synthesis as described herein. The subunit is attached to the solid support, or to a growing oligomer chain on the solid support, via its 5' exocyclic carbon. "Base-protected" refers to protection of the base-pairing groups, e.g. purine or pyrimidine bases, on the morpholino subunits with protecting groups suitable to prevent reaction or interference of the base-pairing groups during stepwise oligomer synthesis.

An "activated phosphoramidate group" is typically a chlorophosphoramidate group, having substitution at nitrogen which is desired in the eventual phosphoramidate linkage in the oligomer. An example is (dimethylamino)chlorophosphoramidate, i.e. -O-P(=O)(NMe₂)Cl.

The terms "charged", "uncharged", "cationic" and "anionic" as used herein refer to the predominant state of a chemical moiety at near-neutral pH, e.g. about 6 to 8. Preferably, the term refers to the predominant state of the chemical moiety at physiological pH, i.e. about 7.4.

"Lower alkyl" refers to an alkyl radical of one to six carbon atoms, as exemplified by methyl, ethyl, n-butyl, i-butyl, t-butyl, isoamyl, n-pentyl, and isopentyl. In selected embodiments, a "lower alkyl" group has one to four carbon atoms, or 1-2 carbon atoms; i.e. methyl or ethyl. Analogously, "lower alkenyl" refers to an alkenyl radical of two to six, preferably three or four, carbon atoms, as exemplified by allyl and butenyl.

### II. Base Protection in PMO Synthesis

Due to the specific challenges of the morpholino chemistry, a base protecting group must fill several requirements. The protecting group should be readily introduced onto the heterocyclic moiety and thereafter be stable to subunit activation and purification conditions, and solid phase synthesis. The protecting group should not be reactive with the morpholino amine moiety of the growing chain, and should allow the activated morpholino subunit to couple cleanly with the growing oligomer chain. The protecting group should be cleaved, preferably by ammonia, without introducing new impurities. Finally, it should result in crystalline subunit derivatives, in order to avoid the need for chromatographic purification prior to activation.

As described below and in the comparative Examples, protecting groups reported in the literature for doubly protected guanosines, as used for nucleic acid synthesis, did not adequately meet these criteria. Thus, a new protecting strategy was required for morpholino G subunits. As described below, use of the 4-(pivaloyloxy)benzyloxy group at 06 was found to me*et al*I of the above criteria.

### A. 06 Protecting Groups: Comparative Data

### A1. 4-nitrophenethyl ether (NPE)

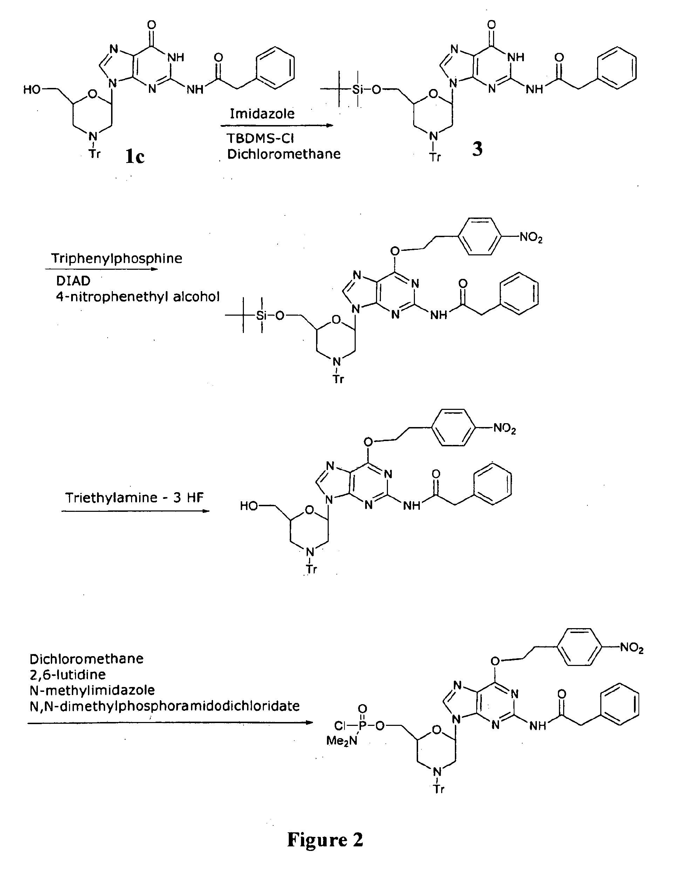

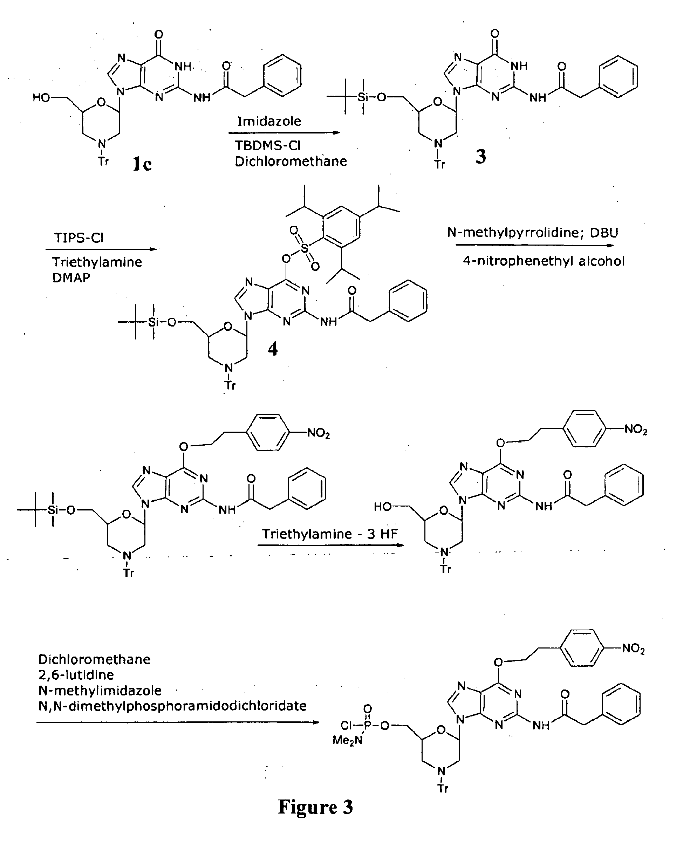

This derivative was prepared as shown in Figure 2 (Mitsunobu 1981) or Figure 3 (Jones *et al*. 1982B). While the crude 06 protected subunit could be prepared in reasonable yield, the compound was not readily crystalline and could be adequately purified only by silica gel chromatography, which is undesirable for large-scale production. After testing an extensive range of reslurrying and/or recrystallization conditions, it was found that butoxyethanol-containing solvent combinations could, with some difficulty, crystallize the material. However, excess butoxyethanol could not be removed from the final product, as the compound likely crystallized as a solvate. The presence of excess alcoholic solvent would not be acceptable in the activation reaction.

The NPE group is cleaved with strong base via a β-elimination mechanism. These conditions tend to generate the reactive by-product 4-nitrostyrene, which can then react with reactive sites on the oligomer. While various scavenging agents (e.g. thiols and 1,3-dicarbonyl compounds) were introduced into the deprotection mixture in an attempt to prevent trapping of the by-product by the oligomer, none were completely successful in eliminating this internal return problem. Even after purification, oligomers prepared with this subunit had a yellow tint.

### A2. Phenylsulfonylethyl (PSE) and Methylsulfonylethyl (MSE)

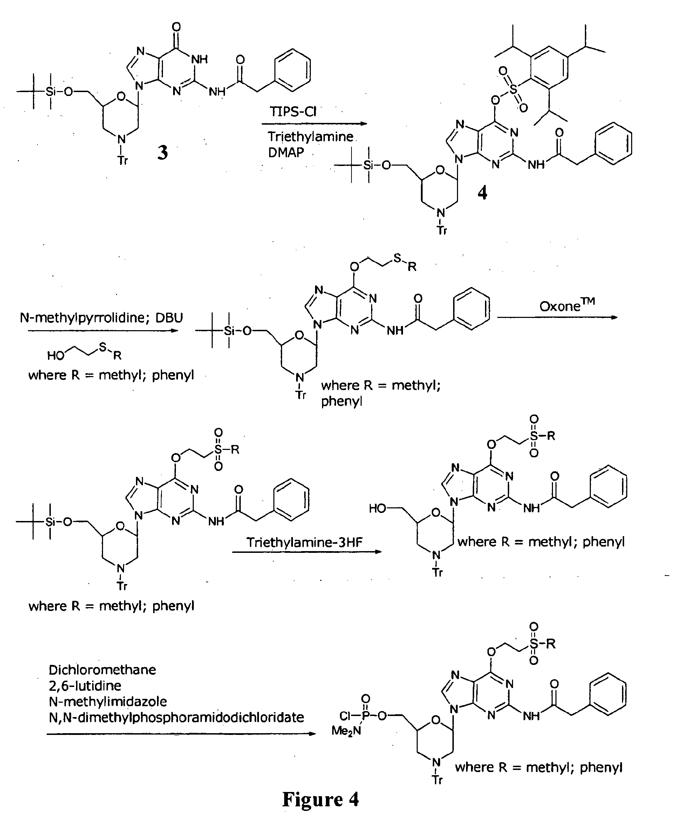

These groups were introduced via the corresponding 2-thioethanol derivatives (Jones *et al.* 1982A, 1982B), as shown in Figure 4. However, no successful crystallization procedure could be found for the resulting subunits.

Like the NPE group, above, these groups are cleaved via a β-elimination mechanism. After incorporation into an oligomer, these derivatives gave the same problems seen with the NPE group; that is, internal return of the reactive alkene by-product formed during deprotection.

### A3. Trimethylsilylethyl ether

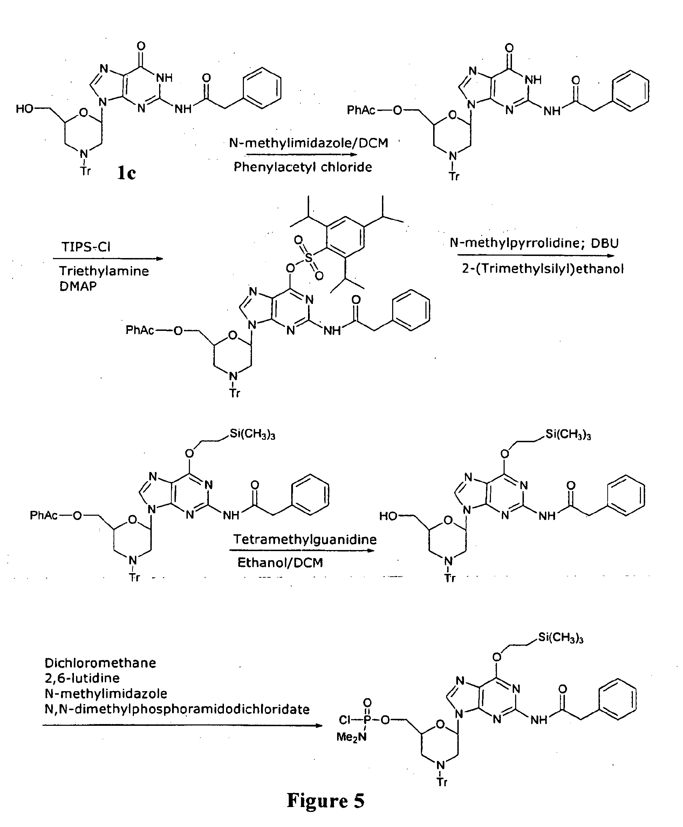

As reported by Jones (Jones *et al.* 1982B), an 06-TMSE-modified morpholino guanine subunit was prepared as shown in Figure 5, but it was not stable during oligomer synthesis. Oligomers made with this subunit showed a range of by-products similar to those made from 06-unprotected G subunits.

### A4. Phenyl ether

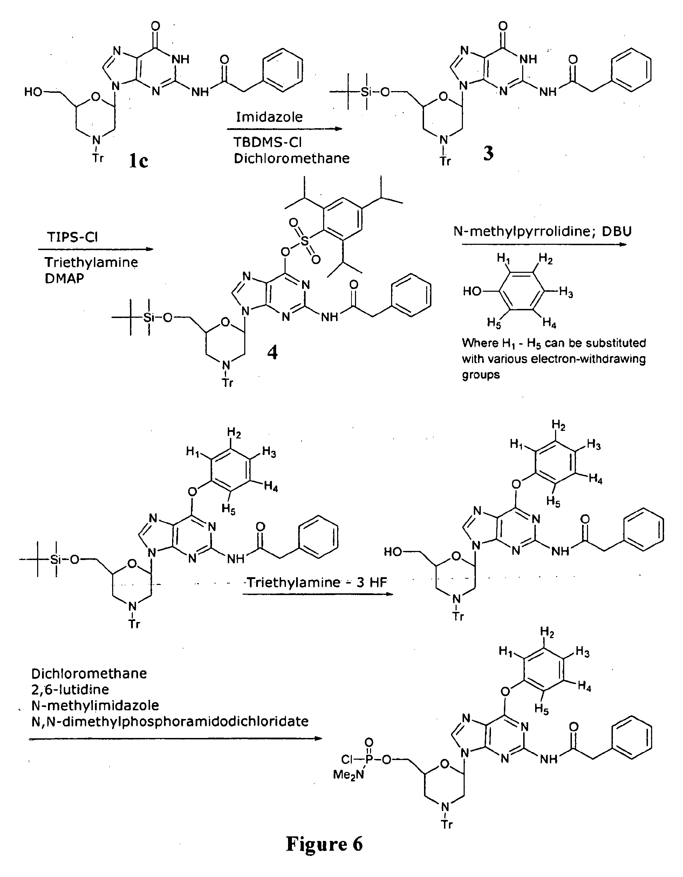

Morpholino guanine subunits with 06-phenyl substitution (Figure 6) were prepared according to the procedure of Reese *et al*. (1981, 1984). The derivatives included unsubstituted phenyl, 2,5-dichlorophenyl, pentafluorophenyl, and 3-fluorophenyl. Such subunits could be incorporated into PMO, but deprotection with the usual reagents, such as 2-nitrobenzaldehyde oxime and strong base, could not be carried to completion without degradation of the oligomer.

### A5. Carbamate

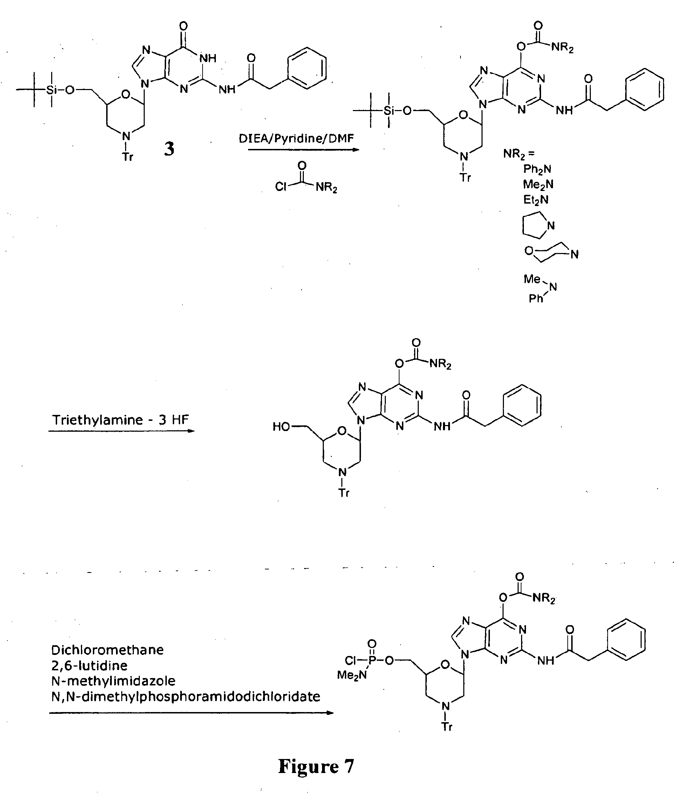

Several 06-carbamate derivatives were synthesized, according to the procedure of Hata *et al.* 1983 (Figure 7). Use of these derivatives in oligomer synthesis gave varying results depending on the derivative used. For the more labile species, such as the diphenyl carbamoyl analog, transfer of the protecting group to the 3'-nitrogen of the growing chain was noted during the coupling step of solid phase synthesis, resulting in truncated oligomers containing a 3'-diphenylcarbamoyl moiety. In addition, the 06-carbamates have two possible sites of reaction with ammonia. While the more reactive moieties such as the diphenylcarbamoyl group gave relatively selective attack at the carbonyl, the more stable dimethyl and pyrrolidinyl carbamates showed significant competing reaction of ammonia at the C6 position, with conversion to diaminopurine.

### B. 4-(Pivaloyloxy)benzyloxy Protecting Group

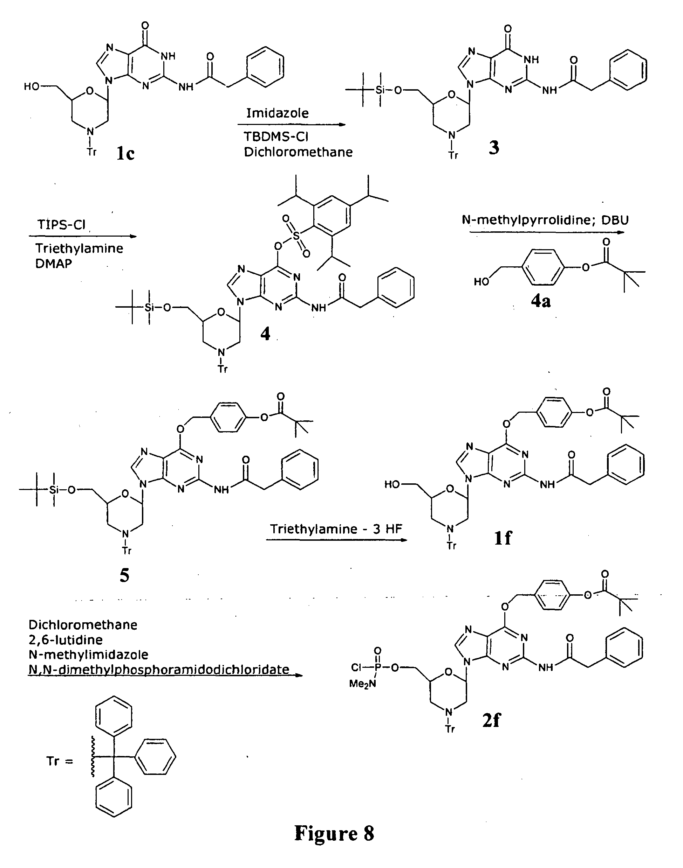

4-(Pivaloyloxy)benzyloxy alcohol (**4a**, Figure 8) was introduced into the morpholino guanine subunit via an efficient, high-yielding synthesis. The subunit prior to activation (compound **1f** in Figures 1 and 8) can be synthesized and reproducibly isolated at large scale without chromatographic purification, and it can be crystallized from a variety of solvents (e.g. THF/water, THF/heptane, acetonitrile, various ester/hydrocarbon mixtures). Ten batches of this subunit made at the 50-200 gallon scale (batch size: 8 - 27 kg of compound **1c**) gave an average yield of 65% of product, having a purity (by HPLC) of 97.6% to 99.2%.

The subunit is converted to activated subunit (i.e., conversion to the 5'-chlorophosphoramidate compound) much more cleanly than mono-protected G, and it can be more easily purified by silica gel chromatography. At scale, overall yield from compound **1f** to compound **2f** (Fig. 1) is approximately 50%.

The POB protecting group may be employed with other combinations of protecting groups for the N2 and morpholino ring nitrogens. Suitable N2 protecting groups include phenylacetyl (as illustrated in Fig. 8) as well as acetyl, propionyl, isobutyryl, and phenoxyacetyl. Trityl species suitable for morpholino ring nitrogen protection between coupling steps include unsubstituted trityl, 4-methyl-, 4,4'-dimethyl-, and 4,4',4"-trimethyltrityl, and 4-methoxytrityl.

Other acyl protecting groups can also be used in place of pivaloyl for the phenol moiety of the POB group. Suitable alternatives include N,N-dimethylcarbamoyl and benzoyl.

During PMO synthesis, no products are seen wherein the pivaloyl group has become attached to the 3'-terminus of smaller fragments of the full length PMO, a side reaction common to the O6-carbamates discussed above. The only notable side product detected was a PMO containing a phenolic residue, resulting from reaction with the deprotection by-product quinone methide. However, this by-product could be reduced to trace levels by sufficient dilution of the ammoniacal deprotection solution. In addition, it is easily removed by virtue of strong binding of the phenolic residue to the polymeric resins used for strong anion exchange chromatography. In general, the overall yield of purified PMO is greatly increased, as seen in Table 1.

The improvement in PMO production fostered by the POB protected guanine group is most evident in the purification following PMO solid phase synthesis, where the difficulty in removing diaminopurine and related byproducts can lead to severe loss during strong anion exchange (SAX) chromatography. For example, crude purities for AVI-4126 prepared with CPM and MPG (mono-protected guanine subunit, **2c**) are in the 68-73% range, which calculates to approximately 58% crude yield of the PMO. During the Trityl-On and Trityl-Off purifications, significant material is lost to obtain pure product, and the overall recovery from the chromatography is 52%. For the AVI-4126 made using CYTFA and DPG (di-protected guanine subunit), the crude purities are 70-75%, with comparable N-1 levels by mass spectrometry (indicating that detritylation efficiencies of CYTFA and CPM reagents are approximately equivalent) and crude yields of about 61%. However, application of the usual purification methods recovers 80% of the PMO from the crude mixture.

**Table 1.**

| PMO AVI- | SEQ ID NO: | Sequence | Detritylation reagent¹ | Guanine Monomer | Scale² | Yield |
|---|---|---|---|---|---|---|
| | | | | | | |
| 4126 | 1 | ACGTTGAGGGGCATCGTCGC | CAA | 2c | 54 g³ | 18% |
| 4557 | 2 | CTGGGATGAGAGCCATCACT | CAA | 2c | 24 g⁴ | 18% |
| " | " | " | CAA | 2c | 48 g⁵ | 15% |
| | | | | | | |
| 4126 | 1 | ACGTTGAGGGGCATCGTCGC | CPM | 2c | 25 g | 25% |
| " | " | " | CPM | 2c | 25 g | 27% |
| " | " | " | CPM | 2c | 25 g | 30% |
| 4020 | 3 | CTTAGTCATCGAGATCTTCGTG | CPM | 2c | 30 g | 32% |
| | | | | | | |
| 4126 | 1 | ACGTTGAGGGGCATCGTCGC | CYTFA | 2f | 25 g | 49% |
| 4065 | 4 | GTGCTCATGGTGCACGGTC⁶ | CYTFA | 2f | 120 g | 46% |
| " | " | " | CYTFA | 2f | 120 g | 49% |
| " | " | " | CYTFA | 2f | 120 g | 50% |

| | | | | | | |
|---|---|---|---|---|---|---|
| Syntheses were performed in accordance with methods described in co-owned US application US 2009/0088562 A1 using the modifications indicated in the table; see Examples 3-6 below. All PMO have a 5"-"tail" and are unsubstituted at the 3'-terminus. ¹CAA = 11% Cyanoacetic acid (w/w) in a mixture of 20% acetonitrile/DCM (v/v); CPM = 2% 3-Chloropyridinum methanesulfonate (w/v) and 0.9% ethanol (v/v) in 20% trifluoroethanol/DCM (v/v); CYTFA = 2% 3-Cyanopyridinum trifluoroacetate (w/v) and 0.9% ethanol (v/v) in 20% trifluoroethanol/DCM (v/v). ²Scale is weight of starting resin in grams. Resin loading is 480-520 micromoles/g ³Combined output of 4x12 g and 1x8 g runs. ⁴Combined output of 2x12 g runs. ⁵Combined output of 4x12 g runs. ⁶Addition of the final C subunit was performed with an activated morpholino C subunit with 4-methoxytrityl protection on the morpholino nitrogen. | | | | | | |

Thus, use of the doubly protected MoG monomer of the invention provides a method of synthesizing a morpholino oligomer in increased purified yield relative to prior art methods, and particularly in comparison to purified yields observed when a monoprotected MoG monomer, or other protected MoG monomer not of the invention, is employed. In particular, the method preferably generates a reduced level of diaminopurine species than would be obtained using a MoG monomer not of the invention.

### III. Doubly Protected Guanine Moroholino Subunits

The doubly protected guanine (DPG) morpholino subunits of the invention have the structure 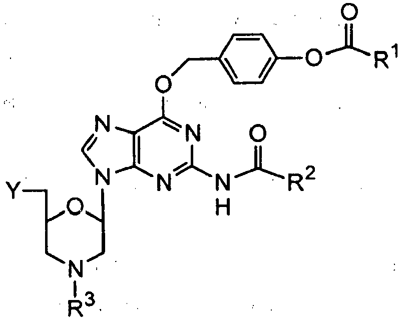 where
R¹ is selected from the group consisting of lower alkyl, di(lower alkyl)amino, and phenyl;
R² is selected from the group consisting of lower alkyl, monocyclic arylmethyl, and monocyclic (aryloxy)methyl;
R³ is selected from the group consisting of triarylmethyl and hydrogen; and
Y is selected from the group consisting of: a protected or unprotected hydroxyl or amino group; a chlorophosphoramidate group; and a phosphorodiamidate linkage to the ring nitrogen of a further morpholino compound or a morpholino oligomer.

In selected embodiments, Y is a protected or unprotected hydroxyl group (as in the pre-activated monomer) or a chlorophosphoramidate group (as in the activated monomer). Preferred protecting groups for the hydroxyl group include trialkylsilyl groups, such as tert-butyldimethylsilyl (TBDMS).

Embodiments in which Y is a phosphorodiamidate linkage to the ring nitrogen of a further morpholino compound, or a phosphorodiamidate linkage to a morpholino oligomer, refer to species formed during the synthesis of a morpholino oligomer, prior to base deprotection.

As discussed below, the substituents on the chlorophosphoramidate group (in the activated monomer) can vary depending on the specific phosphorodiamidate linkage desire.

The invention also provides, correspondingly, a method of synthesizing a morpholino oligomer, the method comprising:
(a) reacting a solid-phase-supported morpholino subunit, having an unprotected ring nitrogen, with a base-protected morpholino subunit monomer, having a triarylmethyl-protected ring nitrogen and an activated phosphoramidate group on a 5'-exocyclic carbon, thereby forming a phosphorodiamidate linkage between said 5'-exocyclic carbon and said unprotected ring nitrogen;
(b) deprotecting said protected ring nitrogen, to form an unprotected ring nitrogen; and
(c) repeating steps (a) and (b) one or more times with further base-protected morpholino subunit monomers;
wherein at least one of said base-protected morpholino subunit monomers is a doubly protected guanine morpholino compound having the structure: 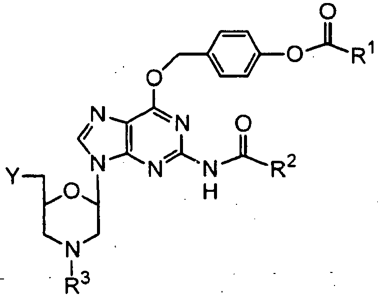 wherein
R¹ is selected from the group consisting of lower alkyl, di(lower alkyl)amino, and phenyl;
R² is selected from the group consisting of lower alkyl, monocyclic arylmethyl, and monocyclic (aryloxy)methyl;
R³ is selected from the group consisting of triarylmethyl and hydrogen; and
Y is a chlorophosphoramidate group.

Preferred triarylmethyl protecting groups for the morpholino ring nitrogen (R³) include trityl (triphenylmethyl), 4-methoxytrityl, 4-methyltrityl, 4,4'-dimethyltrityl, and 4,4',4"-trimethyltrityl.

The R¹ substituent on the 06 protecting group is preferably C₁ to C₄ alkyl, especially -C(CH₃)₃ (tert-butyl), as in the 4-(pivaloyloxy)benzyloxy (POB) group. However, R¹ can also be di(lower alkyl)amino, such as dimethylamino, or phenyl.

As noted above, substitution of the chlorophosphoramidate group Y in "activated" monomers varies depending on the structure of the desired phosphorodiamidate linkage. For preparation of the "standard" uncharged PMO linkage 5'-O-P(=O)(-N(CH₃)₂) -3' (as shown in Formula **II** above where R is methyl), the chlorophosphoramidate group Y is 5'-O-P(=O)Cl-NR₂ (see e.g. compound 2f, Figure 8).

As described in co-owned application filed May 10, 2007, advantageous properties can be obtained by preparing PMOs having cationic as well as neutral intersubunit linkages. In such oligomers, at least one intersubunit linkage between two consecutive morpholino ring structures contains a pendant cationic group. The pendant group bears a distal nitrogen atom that can bear a positive charge at neutral or near-neutral (e.g. physiological) pH.

For preparation of such linkages, the chlorophosphoramidate group Y in the subunit monomers of the invention may have one of the following structures: 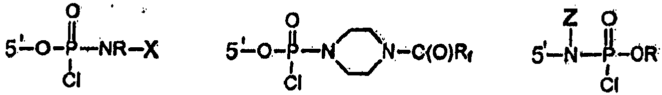
where R is lower alkyl, such as methyl or ethyl;
X = -R⁴-NHC(=O)R_{f}, where R⁴ is bivatent alkyl or oligo PEG, and R_{f} is fully or partially fluorinated methyl, ethyl, or isopropyl; and
Z = X as defined above or lower alkyl, Note that the Z-containing group results in a 5'-amine containing linkage.

The therm "oligo PEG" refers to a group such as -(CH₂-CH₂-O)ₙ-CH₂-CH₂-, where n is typically 1 to 3, and "bivalent alkyl" is typically C₂ to C₈ alkyl,

Following preparation of oligomers using monomers having,such activated chlorophosphoramidate groups, the C(=O)R_{f} protecting groups are removed from the terminal nitrogen atoms, which may be further modified, e.g. to form terminal guanidinyl groups, as described in co-owned application US 2009/0088562 A1.

### IV. Improved Conditions for Deprotection of the Morpholino Ring Nitrogen in PMO Synthesis

As noted above, deprotection of the morpholino ring nitrogen, which is typically protected by a triarylmethyl group such as trityl, in PMO synthesis, must be complete enough at each step to minimize N-1 deletion species. However, studies in support of the invention showed that reagents used in the prior art for this purpose caused an undesirable amount of backbone hydrolysis (see Fig. 1) and degradation. Therefore, efficient deprotecting reagents which at the same time minimized such hydrolysis were sought.

A simple assay was used to test the efficiency of various reagents in deprotection (typically detritylation) ofN-protected morpholino subunits. A model compound, the tritylated moC^{Bz} (i.e. benzoyl-protected cytosine morpholino) subunit shown below, is dissolved in the detritylation solution to be investigated. At various timepoints (e.g. 1, 2, 4 min), an aliquot was quenched and analyzed by TLC or HPLC for completion of morpholino nitrogen deprotection. Generally, for prediction of effective detritylation during solid phase PMO synthesis, this model reaction should be complete within about 2 minutes at room temperature.

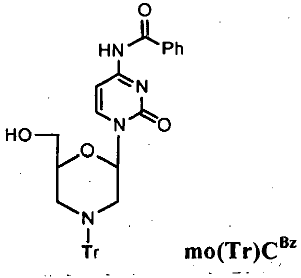

Using this assay and further experimentation, it was determined that various pyridinium salts of strong acids in mixtures of trifluoroethanol (TFE) and dichloromethane (DCM) are excellent catalysts for removing the triarylmethyl protecting group, e.g. a trityl group, from the morpholino nitrogen during solid phase PMO synthesis.

A minimum amount of TFE (~10% v/v or greater) is preferred for reasonable reaction rates and solubilization of the pyridinium salts. Because TFE alone does not swell naked polystyrene, mixtures with DCM (dichloromethane) are preferred, especially in the early cycles of PMO synthesis. Preferred solvent compositions include 10 to 75% TFE.

The use of the TFE solvent is believed to enhance the selectivity of the detritylation reaction over amidate formation (hydrolysis) and phosphorodiamidate (PDA) cleavage, described above, by addressing the differing mechanisms of PDA cleavage and detritylation. TFE is a potent hydrogen bonding solvent and decreases the reactivity of nucleophiles in solution; therefore, it is believed to slow the attack on phosphorus necessary for P-N bond cleavage. TFE also promotes SN1 type solvolysis reactions. The solvolytic character of amine detritylation reactions with TFE is evidenced by the yellow color of detritylation reaction mixtures and the orangish color of demethoxytritylation reaction mixtures. Therefore, increasing TFE concentration is believed to both suppress nucleophilic attack on the PDA linkage and promote detritylation.

Unsubstituted pyridinium salts are not sufficiently acidic for optimal deprotection, but the use of pyridinium species containing electron withdrawing groups (EWG) (e.g. halogen, carbonyl, cyano) allows rapid cleavage of the protecting group. Generally at least 2% (w/v) of such a salt in the TFE:DCM solvent is sufficient for rapid detritylation. Preferred levels of the pyridinium salts are 2 to 10% (w/v).

Acids useful in forming the pyridinium salts include sulfonic acids, such as methanesulfonic, trifluoromethanesulfonic, and p-toluenesulfonic acid, trifluoroacetic acid, and hydrochloric acid. Although a carboxylic acid, trifluoroacetic acid does not cap the growing PMO chain if present during the coupling reaction, and its carboxylate is not sufficiently nucleophilic to promote amidate formation. Particularly preferred are trifluoroacetic and especially methanesulfonic acid.

The pyridines useful in forming the pyridinium salts include halogen substituted pyridines, especially the less expensive chloropyridines, of which 3-chloropyridine is preferred, and cyanopyridines, for which 4-cyanopyridine is preferred. The 3- and 4-cyanopyridines are readily available, inexpensive bulk chemicals. In general, the efficacy of the salts correlates inversely with the pKa of the pyridinium species. Pyridines with electron withdrawing groups range in pKa from about 1 to 4 (Fisher *et al.* 1964, Rogne 1970).

Also useful are nicotinic acid derivatives (i.e. esters, such as ethyl nicotinate, and nicotinamide), as well as their ketone and aldehyde congeners. Generally, however, these are less potent reagents than the cyanopyridinium salts.

It will be appreciated that salts of heterocycles other than pyridines can function as selective detritylation reagents under the conditions described, provided the pKa of the protonated form is similar to that of substituted pyridines of the invention. Examples may be found in the many tables of pKa for heterocycles found in the literature (e.g. Albert 1963). Examples include thiazole (pKa 2.53), pyridazine (pKa 2.33), pyrazole (pKa 2.47), triazole (pKa 2.30), and substituted derivatives thereof, especially derivatives substituted with EWG as described above..

Two particularly preferred salts are 3-chloropyridinium methanesulfonate (CPM) and 4-cyanopyridinium trifluoroacetate (CYTFA), and particularly preferred embodiments of detritylation reagents include solutions of 2% (w/v) of CPM or CYTFA in 20% trifluoroethanol/DCM (v/v) containing 0.9% ethanol (v/v). As shown in Table 1 above, use of these reagents resulted in a significant increase in yield over the conventional cyanoacetic acid reagents.

The more acidic CYTFA is found to be slightly more efficient than CPM. However, much of the increase in yield between the CPM and CYTFA reagents in the Table can be attributed to the use of a doubly protected guanine monomer (DPG) in which the 06 position is protected with a 4-(pivaloyloxy)benzyloxy group, as disclosed in the co-owned and concurrently filed provisional application entitled "Improved Synthesis of Morpholino Oligomers using Doubly Protected Guanine Morpholino Subunits". In general, use of the DPG monomer reduces the amount of diaminopurine-containing side products, while the improved detritylation reagents reduce the amount of backbone hydrolyzed or truncated side products.

Thus, this modification provides a method of synthesizing a morpholino oligomer with reduced hydrolysis of phosphorodiamidate linkages in the backbone, and preferably a reduced or equivalent level of N-1 deletion species, relative to prior art methods. In another aspect, the invention provides a method of deprotecting a triarylmethyl-protected morpholino ring nitrogen during synthesis of a morpholino oligomer, with reduced hydrolysis of phosphorodiamidate linkages in the backbone of the morpholino oligomer relative to that observed when cyanoacetic acid is used as the deprotecting reagent. Preferably, the method also provides a reduced or equivalent level of N-1 deletion species than would be observed when cyanoacetic acid is used as the deprotecting reagent.

A useful further modification is the use of a trityl trapping agent, such as a thiol, to shift the reaction equilibrium towards products. The use of thiol trapping agents has been employed for nucleic acid synthesis (Ravikumar et al., U.S. Patent No. 5,510,476). Mercaptoethanol is a readily available, inexpensive agent useful for this purpose. The presence of the hydroxyl group is not critical for trapping, because simple thiols such as benzylmercaptan perform equally well. Alcohols, such as ethanol and butanol, and even water also serve as trapping agents of the trityl cation.

### Examples

### Example 1: Synthesis of N2-PhAc, 06-POB Doubly Protected Morpholino G (DPG) Subunit (See Fig 8)

*Preparation of **3*** (Starting with 35 kg of **1c**): A 100 G reactor is charged with **1c** (35 kg; 1.0 eq), imidazole (5.0 kg; 1.3 eq) and dichloromethane (279 kg). The batch is cooled to 3°C. A 50 G reactor is cooled to 3°C and charged with t-butylchlorodimethylsilane (10.1 kg; 1.2 eq) and dichloromethane (93 kg). The solution in the 50 G reactor is transferred to the 100 G reactor, and the batch is adjusted to 20°C. Upon reaction completion (1-3 hours), methanol (1.8 kg; 1.0 eq) is charged to the 100 G reactor. After 30 minutes, the solution in the 100 G reactor is charged to a 200 G reactor containing pH 3 citrate buffer (376 kg of 1 M citric acid adjusted to pH 3 with solid NaOH). The batch is agitated for 30 minutes, and the layers are separated. The lower organic layer is washed once more with pH 3 citrate buffer, and once with brine solution (287 kg of 2.5% NaCl/water (w:w)). The resulting organic solution is distilled at <35°C until Karl Fischer analysis of the batch shows < 0.05% water. This solution is cooled to 3°C in the 100 G reactor and is used directly in the preparation of compound **4**.

*Preparation of **4***: The 100 G reactor containing the solution of compound **3** is charged with triethylamine (6.8 kg; 1.2 eq), 4-dimethylaminopyridine (0.68 kg; 0.1 eq), and triisopropylbenzenesulfonyl chloride (18.6 kg; 1.1 eq). The batch is warmed to 20°C. Upon reaction completion (3-9 hours), the solution is charged to a 200 G reactor containing pH 4.5 phosphate buffer (228 kg of 1 M KH₂PO₄). The batch is agitated for 30 minutes, and the layers are separated. The lower organic layer is washed with brine (212 kg of 2.5% NaCl/water (w:w)). The resulting organic solution is distilled at <35°C until Karl Fischer analysis of the batch shows < 0.01% water. This solution is cooled to 3°C in the 100 G reactor and is used directly in the preparation of compound **5**.

*Preparation of **4a*** (Starting with 60 kg of 4-hydroxybenzaldehyde): A 750 G reactor is charged with 4-hydroxybenzaldehyde (60 kg; 1.0 eq), toluene (260 kg), and 1-methylimidazole (8.1 kg; 0.2 eq). To this solution is charged a solution of potassium bicarbonate (100 kg; 2.0 eq) in water (400 kg), followed by trimethylacetyl chloride (83 kg; 1.4 eq). This two-phase mixture is agitated at 20°C. Upon reaction completion (1-5 hours), methanol (15.7 kg; 1.0 eq) is charged to the batch. The batch is agitated at 20°C for 1 hour. The layers are separated. To the upper organic layer is charged water (200 kg). The batch is agitated for 30 minutes, and the layers are separated. To the upper organic layer is charged pH 4.5 phosphate buffer (16.5 kg KH₂PO₄ in 242 kg water). The batch is agitated for 30 minutes, and the layers are separated. To the upper organic layer is charged water (200 kg). The batch is agitated for 30 minutes, and the layers are separated. The upper organic layer is distilled under vacuum at <30°C to achieve a batch volume of 200 L. THF (70 kg) is charged to the batch, and the batch is transferred to a 500 G reactor containing Pd/C (9.6 kg; 0.004 eq; 5% Pd/C, 50% wet Johnson Matthey Type A405028-5 or A570129-5). The reactor is initially pressurized to 5 psi H₂ with the agitation set at 50 rpm. Both the pressure and agitation rate are slowly increased as the reaction proceeds, to a maximum of 25 psi H₂ and 90 rpm. Upon reaction completion (8-48 hours), the batch is filtered through a pad of Celite followed by a 0.1 micron inline filter. The Celite is rinsed with toluene (20 kg). To the batch is charged pH 6.5 phosphate buffer solution (2.7 kg KH₂PO₄ and 2.3 kg potassium phosphate, dibasic, trihydrate in 200 kg water). The batch is agitated for 30 minutes, and the layers are separated. The upper organic layer is distilled under vacuum at <30°C to achieve a batch volume of 140 L. Toluene (126 kg) is charged to the batch, and the batch is distilled under vacuum at <30°C to achieve a batch volume of 140 L. The batch is adjusted to 20°C, and transferred to a 500 G reactor containing n-heptane (821 kg) and seed crystals of compound **4a** (100 grams) held at 0°C. The batch is held at 0°C for 1-2 hours. A second portion of seed crystals (100 grams) is added, and the batch is held at 0°C for 1-2 hours. Compound **4a** is isolated by filtration. Yield = 70 - 80% from 4-hydroxybenzaldehyde.

The derivative in which the phenol moiety is protected as its N,N-dimethylcarbamate instead of the pivalate ester is made under conditions similar to **4a**. In order to push to completion the reaction between 4-hydroxybenzaldehyde and dimethylcarbamoyl chloride, the reaction is performed in refluxing dichloromethane in the presence of N-methylimidazole as base and 0.2 eq DMAP as catalyst.

*Preparation of **5***: A 100 G reactor containing the solution of compound **4** is charged with N-methylpyrrolidine (9.5 kg; 2.0 eq dissolved in 23 kg of dichloromethane). After 10 minutes, compound **4a** (14.0 kg; 1.2 eq) is added, followed by 1,8-diazabicyclo[5.4.0]undec-7-ene (10.2 kg; 1.2 eq in 23 kg dichloromethane). The batch is warmed to 20°C. Upon reaction completion (1-9 hours), the solution is diluted with 327 kg of dichloromethane and charged to a 200 G reactor containing pH 4.5 phosphate buffer (334 kg of 1 M KH₂PO₄). The batch is agitated for 30 minutes, and the layers are separated. The lower organic layer is washed once more with pH 4.5 phosphate buffer (111 kg of 1 M KH₂PO₄), then once with brine (212 kg of 2.5% NaCl/water (w:w)). The resulting organic solution is distilled at < 35°C until Karl Fischer analysis of the batch shows < 0.05% water. This solution is used directly in the preparation of compound **1f**.

*Preparation of **1f***: A 100 G reactor containing the solution of compound **5** is charged with triethylamine trihydrofluoride (18.0 kg; 2.0 eq). The batch is agitated at 20°C. Upon reaction completion (4-20 hours), the batch is charged to a 200 G reactor. The 200 G reactor is charged with NaHCO₃ solution (230 kg of a 5% (w:w) solution). The batch is agitated for 30 minutes, and the layers are separated. The lower organic layer is washed once more with NaHCO₃ solution (230 kg of a 5% (w:w) solution), then once with pH 6.5 phosphate buffer (9.3 kg KH₂PO₄ and 14.0 kg K₂HPO₄ in 215 kg water). The resulting organic solution undergoes solvent exchange to THF (to achieve <1% DCM by weight in the batch). The solution is diluted with THF (124 kg) and heated to 60°C. Water (8 kg per kg of compound 1f in solution based on LOE analysis; pre-heated to 60°C) is charged slowly to the THF solution. The solution is slowly cooled to 3°C and held for >4 hours. Crude compound **1f** is isolated by filtration. The crude material is re-dissolved in THF (342 kg) and heated to 60°C. Water (315 kg; pre-heated to 60°C) is charged slowly to the THF solution. The solution is cooled to 3°C and held for >4 hours. Compound **1f** is isolated by filtration. A second recrystallization can be performed to further purify compound **1f** if desired. Yield = 53 - 73% from **1c**.

*Preparation of **2f*** (Starting with 12 kg of **1f**): A 50 G reactor is charged with compound **1f** (12 kg; 1.0 eq), dichloromethane (159 kg), 2,6-lutidine (2.5 kg; 1.6 eq) and 1-methylimidazole (0.36 kg; 0.3 eq). This solution is distilled to achieve a batch volume of 69 L, and cooled to 5°C. N, N-Dimethylphosphoramidodichloridate (3.8 kg; 1.6 eq) is charged to the batch. The batch is adjusted to 20°C. Upon reaction completion (6-16 hours), toluene (78 kg) is charged to the batch. The resulting mixture is distilled at 25°C to achieve a batch volume of 126 L (GC analysis of the batch must show 30-45% DCM by weight), and transferred to a 100 G reactor containing pH 3 citrate buffer (15.4 kg citric acid monohydrate, 1.4 kg NaOH, 80 kg water). The batch is agitated for 10 minutes, and the layers are separated. The lower aqueous layer is sent to waste. The upper organic layer is transferred to the 50 G reactor containing sodium sulfate (8.0 kg). The batch is agitated for 30 minutes, and the sodium sulfate waste cake is removed by filtration. The sodium sulfate cake is rinsed with dichloromethane (16 kg). The resulting product solution is distilled in the 50 G reactor to achieve a batch volume of 53 L (GC analysis of the batch must show 11-15% DCM by weight). The 100 G reactor is charged with heptane (238 kg). The batch in the 50 G reactor is transferred to the 100 G reactor over 2 hours. At the end of the transfer, the batch is held at 20°C for 4-16 hours. The crude compound **6** is collected by filtration. The crude material is charged to the 100 G reactor. To the crude solids is added a solution of toluene (16 kg) and heptane (50 kg). This mixture is agitated for 3 hours and filtered. The reslurry is repeated one or more times. Yield of crude **2f** = 80% from **1f**.

*Purification of Compound 2f* by Silica Gel Chromatography (Starting with ~ 6.5 kg of crude compound **2f**): The "strength" of crude compound **2f** is calculated by correcting the weight of crude material for HPLC purity and volatiles. For this purification step, 5.75 kg of material (corrected for strength) is used per injection on a 50 cm chromatography column. The 50 cm chromatography column is packed with a slurry of heptane/silica gel (51.8 kg of silica gel). The crude material is loaded onto the column as a solution in dichloromethane/2,6-lutidine (15 kg dichloromethane, 0.16 kg 2,6-lutidine). The product is eluted with a two-step gradient of 4-methyl-2-pentanone (MIBK)/heptane/2,6-lutidine (first step is 827 L of 39:61 MIBK:heptane (w:w) with 0.06% 2,6-lutidine (w:w); second step is 1343 L of 73:27 MIBK:heptane (w:w) with 0.06% 2,6-lutidine (w:w)). The approved fraction pool is concentrated via thin-film evaporation to a concentration of 150 g/L. This concentrated pool is precipitated onto 6 volumes of heptane. The purified **2f** is isolated by filtration. Yield of purified **2f** = 50% from **1f**; 65% from crude **2f**.

### Example 2. Preparation of CYTFA Pyridinium Salt Detritylation Solution

To a solution of 4-cyanopyridine (10.1 g; 1.055 eq) in dichloromethane (790 mL) is added trifluoroacetic acid (10.5 g; 1.0 eq) followed by 2,2,2-trifluoroethanol (198 mL) and ethanol (10 mL) and the solution is stirred for 10-30 min.

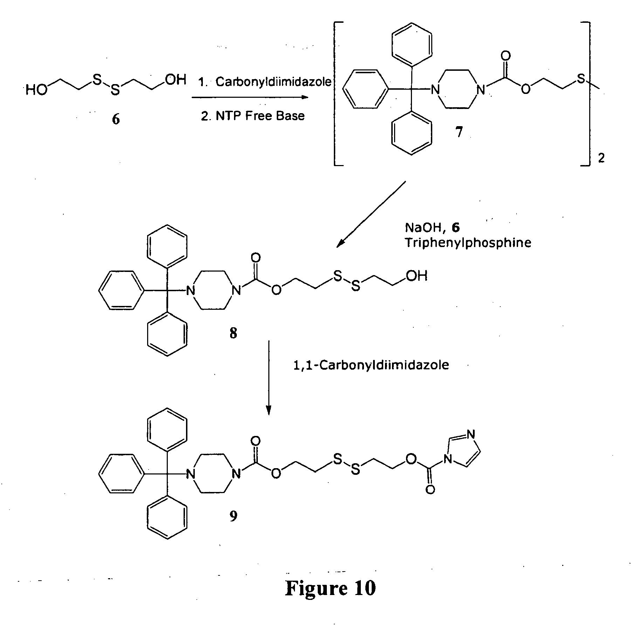

### Example 3: Preparation of Disulfide Anchor (See Fig. 10)

*Preparation of N-trityl piperazine, succinate salt (NTP):* To a cooled solution of piperazine (10 eq) in toluene/methanol-(5:1 toluene/methanol-(v:v); 5 mL/g piperazine) was added slowly a solution of triphenylmethyl (trityl) chloride (1.0 eq) in toluene (5 mL/g trityl chloride). Upon reaction completion (1 - 2 hr), this solution was washed four times with water. To the resulting organic solution was added an aqueous solution of succinic acid (1.1 eq; 13 mL water/g succinic acid). This mixture was stirred for 90 min, and the solid product was collected by filtration. The crude NTP was purified by two reslurries in acetone. Yield = 70%.

*Preparation of symmetrical disulfide 7:* 1,1'-Carbonyldiimidazole (CDI) (12.402 g; 2.2 eq.) was suspended in dichloromethane (5.25 mL/g) and cooled on an ice bath. Hydroxyethyl disulfide **6** (5.36 g; 1 eq.) was dissolved in dichloromethane (10 mL/g) and tetrahydrofuran (1 mL/g). The diol solution was added to the CDI slowly such that the temperature of the mixture stayed below 4 °C for the duration of the reaction. Upon reaction completion (once addition was complete), de-ionized water (93.8 µL, 0.15 eq.) was added to quench the reaction. Independently, N-trityl piperazine, succinate salt (NTP) (32.59 g; 2.1 eq.) was dissolved in toluene (8 mL/g NTP), dichloromethane (2 mL/g NTP), and methanol (2 mL/g NTP). K₂CO₃ (22.09 g; 4.6 eq.) was dissolved in de-ionized water (10 mL/g). The K₂CO₃ solution added to the solution of NTP; the mixture was stirred and then separated into two layers. The cloudy organic layer was distilled to remove 90 grams; the resulting water droplets were separated and acetone (8 mL/g NTP) was added to the organic layer. The solution of CDI activated disulfide diol was added to the solution of the free base and concentrated to 225 mL. Acetone (10 mL/g NTP) was added and the mixture was concentrated to 225 mL. The mixture was heated to reflux and solid began crystallizing out of solution. Upon completion, the reaction mixture was cooled and the solid (7) was isolated by filtration. Yield: 27.92 g; 93.1% (based on weight-based assay).

*Preparation of disulfide alcohol **8**:* 7 (36.00 g; 32.1 mmol; 1 eq.) was suspended in acetone (2.8 mL/g 7). Hydroxyethyl disulfide (78.51 mL; 20 eq.) was added followed by acetone (1.7 mL/g 7). 5% NaOH/methanol (2.85 mL; 0.1 eq.) was added; the pH of the mixture was 10 by pH paper. Triphenylphosphine (8.42 g; 1 eq.) was added followed by acetone (1.1 mL/g 7). All solids went into solution and then product began to crystallize out. After sixteen hr, the reaction mixture was neutralized with acetic acid (2.4 g; 0.2 eq.). The crude product was isolated by filtration. The crude solid 8 was subjected to two refluxing acetone reslurries (5 mL/g 7).

After filtration the crude product was suspended in dichloromethane (7.25 mL/g 7). The mixture was heated until a clear solution formed (35 °C). The solution was extracted five times with an equal volume of de-ionized water and the final organic layer was concentrated to 155 mL. Dichloromethane was added (4.3 mL/g 7), and the solution was again concentrated to 155 mL. CDI (9.17 g; 1.1 eq.) was added and the mixture was stirred at room temperature. Upon reaction completion (∼20 min) the reaction mixture was washed twice with an equal volume of de-ionized water, then ethylbenzene (2.1 mL/g 7) was added. The solution was concentrated to 65.2 g, reducing the dichloromethane in the solution to 0.17%, and stirred on an ice bath to crystallize the product. The product 9 was isolated by filtration. Yield: 44%.

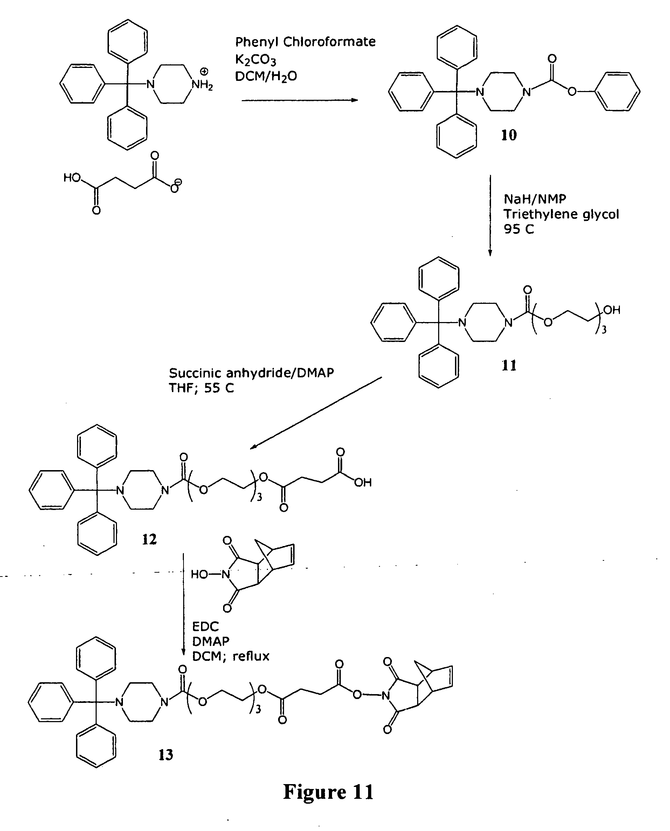

### Example 4: Triethylene glycol Tail (See Fig. 11)

*Preparation of trityl piperazine phenyl carbamate* **10:** To a cooled suspension of NTP in dichloromethane (6 mL/g NTP) was added a solution of potassium carbonate (3.2 eq) in water (4 mL/g potassium carbonate). To this two-phase mixture was slowly added a solution of phenyl chloroformate (1.03 eq) in dichloromethane (2 g/g phenyl chloroformate). The reaction mixture was warmed to 20 °C. Upon reaction completion (1-2 hr), the layers were separated. The organic layer was washed with water, and dried over anhydrous potassium carbonate. The product **10** was isolated by crystallization from acetonitrile. Yield = 80%

*Preparation of carbamate alcohol* **11**: Sodium hydride (1.2 eq) was suspended in 1-methyl-2-pyrrolidinone (32 mL/g sodium hydride). To this suspension were added triethylene glycol (10.0 eq) and compound **10** (1.0 eq). The resulting slurry was heated to 95 °C. Upon reaction completion (1-2 hr), the mixture was cooled to 20 °C. To this mixture was added 30% dichloromethane/methyl tert-butyl ether (v:v) and water. The product-containing organic layer was washed successively with aqueous NaOH, aqueous succinic acid, and saturated aqueous sodium chloride. The product **11** was isolated by crystallization from dichloromethane/methyl tert-butyl ether/heptane. Yield = 90%.

*Preparation of Tail acid* **12:** To a solution of compound **11** in tetrahydrofuran (7 mL/g **11**) was added succinic anhydride (2.0 eq) and DMAP (0.5 eq). The mixture was heated to 50 °C. Upon reaction completion (5 hr), the mixture was cooled to 20 °C and adjusted to pH 8.5 with aqueous NaHCO₃. Methyl tert-butyl ether was added, and the product was extracted into the aqueous layer. Dichloromethane was added, and the mixture was adjusted to pH 3 with aqueous citric acid. The product-containing organic layer was washed with a mixture of pH=3 citrate buffer and saturated aqueous sodium chloride. This DCM solution of **12** was used without isolation in the preparation of compound **13**.

*Preparation of* **13:** To the solution of compound **12** was added N-hydroxy-5-norbomene-2,3-dicarboxylic acid imide (HONB) (1.02 eq), 4-dimethylaminopyridine (DMAP) (0.34 eq), and then 1-(3-dimethylaminopropyl)-N'-ethylcarbodiimide hydrochloride (EDC) (1.1 eq). The mixture was heated to 55 °C. Upon reaction completion (4-5 hr), the mixture was cooled to 20 °C and washed successively with 1:1 0.2 M citric acid/brine and brine. The dichloromethane solution underwent solvent exchange to acetone and then to N,N-dimethylformamide, and the product was isolated by precipitation from acetone/ N,N-dimethylformamide into saturated aqueous sodium chloride. The crude product was reslurried several times in water to remove residual N,N-dimethylformamide and salts. Yield = 70% of **13** from compound **11**. Introduction of the activated "Tail" onto the disulfide anchor-resin was performed in NMP by the procedure used for incorporation of the subunits during solid phase synthesis.

### Example 5: Preparation of the Solid Support for Synthesis of Morpholino Oligomers

### Example 5a: Preparation of Aminomethylpolystyrene-disulfide resin

This procedure was performed in a silanized, jacketed peptide vessel (custom made by ChemGlass, NJ, USA) with a coarse porosity (40-60 µm) glass frit, overhead stirrer, and 3-way Teflon stopcock to allow N₂ to bubble up through the frit or a vacuum extraction. Temperature control was achieved in the reaction vessel by a circulating water bath.

The resin treatment/wash steps in the following procedure consist of two basic operations: resin fluidization and solvent/solution extraction. For resin fluidization, the stopcock was positioned to allow N₂ flow up through the frit and the specified resin treatment/wash was added to the reactor and allowed to permeate and completely wet the resin. Mixing was then started and the resin slurry mixed for the specified time. For solvent/solution extraction, mixing and N₂ flow were stopped and the vacuum pump was started and then the stopcock was positioned to allow evacuation of resin treatment/wash to waste. All resin treatment/wash volumes were 15 mL/g of resin unless noted otherwise.

To aminomethylpolystyrene resin (100-200 mesh; ~1.0 mmol/g N₂ substitution; 75 g, 1 eq, Polymer Labs, UK, part #1464-X799) in a silanized, jacketed peptide vessel was added 1-methyl-2-pyrrolidinone (NMP; 20 ml/g resin) and the resin was allowed to swell with mixing for 1-2 hr. Following evacuation of the swell solvent, the resin was washed with dichloromethane (2 x 1-2 min), 5% diisopropylethylamine in 25% isopropanol/dichloromethane (2 x 3-4 min) and dichloromethane (2 x 1-2 min). After evacuation of the final wash, the resin was fluidized with a solution of disulfide anchor **9** in 1-methyl-2-pyrrolidinone (0.17 M; 15 mL/g resin, ~2.5 eq) and the resin/reagent mixture was heated at 45°C for 60 hr. On reaction completion, heating was discontinued and the anchor solution was evacuated and the resin washed with 1-methyl-2-pyrrolidinone (4 x 3-4 min) and dichloromethane (6 x 1-2 min). The resin was treated with a solution of 10% (v/v) diethyl dicarbonate in dichloromethane (16 mL/g; 2 x 5-6 min) and then washed with dichloromethane (6 x 1-2 min). The resin **14** was dried under a N₂ stream for 1-3 hr and then under vacuum to constant weight (± 2%). Yield: 110-150% of the original resin weight.

### Example 5b: Determination of the Loading of Aminomethylpolystyrene-disulfide resin

The loading of the resin (number of potentially available reactive sites) is determined by a spectrometric assay for the number of triphenylmethyl (trityl) groups per gram of resin.

A known weight of dried resin (25 ± 3 mg) is transferred to a silanized 25 ml volumetric flask and -5 mL of 2% (v/v) trifluoroacetic acid in dichloromethane is added. The contents are mixed by gentle swirling and then allowed to stand for 30 min. The volume is brought up to 25 mL with additional 2% (v/v) trifluoroacetic acid in dichloromethane and the contents thoroughly mixed. Using a positive displacement pipette, an aliquot of the trityl-containing solution (500 µL) is transferred to a 10 mL volumetric flask and the volume brought up to 10 mL with methanesulfonic acid.

The trityl cation content in the final solution is measured by UV absorbance at 431.7 nm and the resin loading calculated in trityl groups per gram resin (µmol/g) using the appropriate volumes, dilutions, extinction coefficient (ε: 41 µmol⁻¹cm⁻¹) and resin weight. The assay is performed in triplicate and an average loading calculated.

The resin loading procedure in this example will provide resin with a loading of approximately 500 µmol/g. A loading of 300-400 in µmol/g was obtained if the disulfide anchor incorporation step is performed for 24 hr at room temperature.

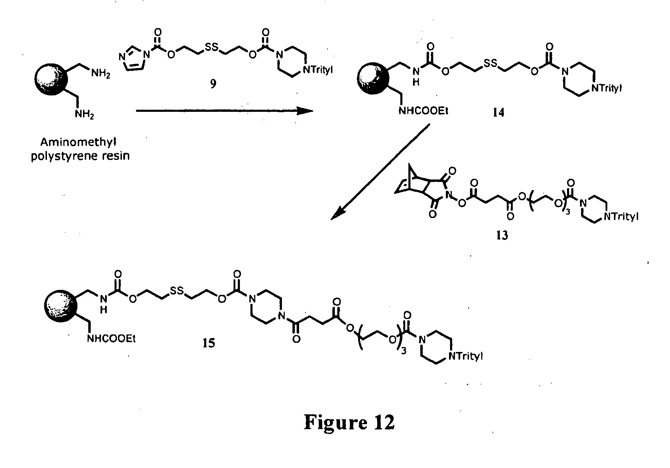

### Example 5c: Tail loading (See Fig. 12)

Using the same setup and volumes as for the preparation of aminomethylpolystyrene-disulfide resin, the Tail can be introduced into the molecule. For the coupling step, a solution of **13** (0.2 M) in NMP containing 4-ethylmorpholine (NEM, 0.4 M) was used instead of the disulfide anchor solution. After 2 hr at 45 °C, the resin **15** was washed twice with 5% diisopropylethylamine in 25% isopropanol/dichloromethane and once with DCM. To the resin was added a solution of benzoic anhydride (0.4 M) and NEM (0.4 M). After 25 min, the reactor jacket was cooled to room temperature, and the resin washed twice with 5% diisopropylethylamine in 25% isopropanol/dichloromethane and eight times with DCM. The resin **15** was filtered and dried under high vacuum. The loading for resin **15** is defined to be the loading of the original aminomethylpolystyrene-disulfide resin **14** used in the Tail loading.

### Example 6: Synthesis of Morpholino Oligomers

### Example 6a: Solid Phase Synthesis

Protected oligomers were prepared manually by solid phase oligomer synthesis on aminomethylpolystyrene-disulfide resin (~500 µmol/g loading) at 10 g scale (starting resin weight). Solutions used were as follows:
Detritylation solutions: CAA = 11% Cyanoacetic acid (w/w) in a mixture of 20% acetonitrile/DCM (v/v);
CPM = 2% 3-Chloropyridinum methanesulfonate (w/v) and 0.9% ethanol (v/v) in 20% trifluoroethanol/DCM (v/v);
CYTFA = 2% 3-Cyanopyridinum trifluoroacetate (w/v) and 0.9% ethanol (v/v) in 20%trifluoroethanol/DCM (v/v).
Neutralization solution: 5% diisopropylethylamine in 25% isopropanol/dichloromethane;
Coupling solutions: 0.165 M (for **2f** (DPG), **2c**, and **2d** or other T subunits) or 0.18 M (for **2a** and **2b** or other A/C subunits) activated Morpholino Subunit and 0.4 M N-ethylmorpholine in 1,3-dimethylimidazolidinone (DMI).
Activated MPG (**2c**) was prepared as in Summerton *et al*. (1993).

After transfer of the resin to the synthesis reactor and prior to initiating synthesis cycles, 1-methyl-2-pyrrolidinone (NMP, 20 mL/g resin) was added and allowed to sit for 1-2 hrs. After washing 2 times with dichloromethane (10 mL/g resin), the following synthesis cycle was used with addition of the appropriate coupling solution of activated Morpholino Subunit of the desired base and desired linkage type at each cycle to give the proper sequence.

| **Step** | **Volume (mL/g of starting resin)*** | **Time (min)** |
|---|---|---|
| DCM | 10-30 | 1-2 |
| DCM | 10-30 | 1-2 |
| Detritylation A | 10-30 | 2-3 |
| Detritylation A | 10-30 | 2-3 |
| Detritylation A | 10-30 | 2-3 |
| Detritylation A | 10-30 | 2-3 |
| Detritylation A | 10-30 | 2-3 |
| Neutralization A | 10-30 | 3-4 |
| Neutralization A | 10-30 | 3-4 |
| Neutralization A | 10-30 | 3-4 |
| Neutralization A | 10-30 | 3-4 |
| DCM | 10-30 | 1-2 |
| DCM | 10-30 | 1-2 |
| Coupling | 7-12 | 90 |
| Neutralization A | 10-30 | 1-2 |
| Neutralization A | 10-30 | 1-2 |
| Neutralization A | 10-30 | 1-2 |
| Neutralization A | 10-30 | 1-2 |
| DCM | 10-30 | 1-2 |
| * Wash volumes are incremented to account for resin swelling; volume is 10 mL/g of actual resin volume at each cycle | | |
| ** Coupling volumes are sufficient to maintain good mixing and are incremented to account for resin swelling | | |

After incorporation of the final subunit, a final cycle (methoxytritylation) was performed with 0.32 M 4-methoxytriphenylmethyl chloride and 0.4 M N-ethylmorpholine in DMI. After methoxytritylation, the resin was washed 8 times with NMP and then treated with cleavage solution consisting of 0.1 M 1,4-dithiothreitol (DTT) and 0.73 M triethylamine in NMP (27 mL/g starting resin) for 30 min. After collection of the protected oligomer solution, the resin (significantly reduced in volume) was washed with two additional portions of cleavage solution (13 mL/g starting resin for 15 min each) and the washes were combined with the bulk solution. To the protected oligomer solution in an appropriately sized pressure bottle with Teflon plug (Ace Glass, NJ, USA) was added concentrated aqueous ammonia (106 mL/g starting resin, previously cooled to -20°C), the bottle sealed, and the contents mixed by swirling. The bottle was placed in a 45°C oven for 16-20 hr to remove base and backbone protecting groups.

Following ammonolysis, the crude oligomer solution is cooled to room temperature and then diafiltered against 0.28% aqueous ammonia using a PLBC 3kd Regenerated Cellulose membrane (Millipore) to remove solvents and small molecules prior to ion exchange chromatography.

### Example 6b: Purification of Morpholino Oligomers by Anion Exchange Chromatography

The crude oligomer solution obtained from diafiltration is adjusted to pH 11-11.5 and loaded onto a column of ToyoPearl Super-Q 650S anion exchange resin (Tosoh Bioscience). The methoxytritylated oligomer is eluted with a gradient of 5-35% B over 17 column volume (Buffer A: 10 mM sodium hydroxide; Buffer B: 1 M sodium chloride in 10 mM sodium hydroxide) and fractions of acceptable purity (anion exchange HPLC and mass spec) pooled.

### Example 6c: Demethoxytritylation of Morpholino Oligomers

To the pooled fractions from anion exchange chromatography is added acetonitrile (10% by volume) followed by 2 M H₃PO₄ to adjust the pH to 3. The solution is mixed for 45 min and then neutralized with concentrated aqueous ammonia to pH 7. The oligomer solution is diafiltered against 20 mM sodium acetate using a PLBC 3kd Regenerated Cellulose membrane (Millipore) to exchange buffers prior to cation exchange chromatography.

### Example 6d: Purification of Morpholino Oligomers by Cation Exchange Chromatography

The oligomer solution is adjusted to pH 4.5 with acetic acid and loaded onto a column of Source 30S cation exchange resin (GE Healthcare). The oligomer is eluted with a gradient of 0-35% B over 17 column volumes (Buffer A: 20 mM sodium acetate, 25% acetonitrile, pH 4.5; Buffer B: 0.5 M sodium chloride, 20 mM sodium acetate, 25% acetonitrile, pH 4.5) and fractions of acceptable purity (cation exchange HPLC and mass spec) pooled.

## Claims

1. A morpholino compound having the following structure 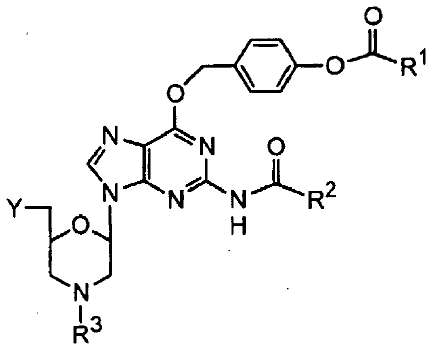 wherein:
R¹ is selected from the group consisting of lower alkyl, di(lower alkyl)amino, and phenyl;
R² is selected from the group consisting of lower alkyl, monocyclic arylmethyl, and
monocyclic (aryloxy)methyl;
R³ is selected from the group consisting of triarylmethyl and hydrogen; and
Y is selected from the group consisting of: a protected or unprotected hydroxyl or amino group; a chlorophosphoramidate group; a phosphorodiamidate linkage to the morpholino ring nitrogen of a further morpholino compound, a phosphorodiamidate linkage to the morpholino ring nitrogen of a morpholino subunit in a morpholino oligomer or a solid support;
wherein the term lower alkyl defines an alkyl radical of one to six carbon atoms;
provided that the compound does not have the following structure: 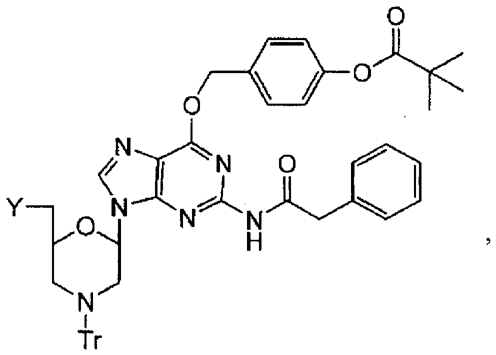 wherein:
Y is -OH, 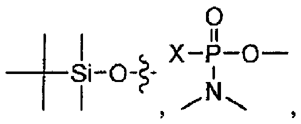 wherein X is Cl, the morpholino ring nitrogen of a further morpholino compound, the morpholino ring nitrogen of a morpholino subunit in a morpholino oligomer or a solid support; and
Tr is trityl.

2. The compound of claim 1, wherein Y is selected from the group consisting of a protected or unprotected hydroxyl group and a chlorophosphoramidate group.

3. The compound of claim 1, wherein Y is a trialkylsilyl-protected hydroxyl group.

4. The compound of claim 1, wherein Y is a chlorophosphoramidate group of the form -OP(=O)-N(CH₃)₂Cl.

5. The compound of claim 1, wherein R³ is selected from trityl, 4-methoxytrityl, 4-methyltrityl, 4,4'-dimethyltrityl, and 4,4',4"-trimethyltrityl.

6. The compound of claim 1, wherein R¹ is lower alkyl, wherein the term lower alkyl defines an alkyl radical of one to six carbon atoms.

7. The compound of claim 6, wherein R¹ is -C(CH₃)₃.

8. The compound of claim 1, wherein R² is benzyl or -CH(CH₃)₂.

9. A method of synthesizing a morpholino oligomer, the method comprising:
(a) reacting a solid-phase-supported morpholino subunit, having an unprotected ring nitrogen, with a first morpholino subunit monomer having a triarylmethyl-protected ring nitrogen and an activated phosphoramidate group on a 5'-exocyclic carbon, thereby forming a phosphorodiamidate linkage between said 5'-exocyclic carbon and said unprotected ring nitrogen;
(b) deprotecting said protected ring nitrogen, to form a product comprising an unprotected ring nitrogen;
(c) optionally reacting the product from step (b) with a further morpholino subunit monomer comprising a triarylmethyl-protected morpholino ring nitrogen and an activated phosphoramidate group on a 5'-exocyclic carbon, thereby forming a phosphoramidate linkage between the 5'-exocyclic carbon of the further morpholino subunit monomer and the unprotected morpholino ring nitrogen of the product from step (b); and
(d) optionally repeating steps (b) and (c) one or more times;
wherein at least one of the first morpholino subunit monomer, the further morpholino subunit monomer or the solid-phase-supported morpholino subunit monomer is a doubly protected guanine morpholino compound having the structure: 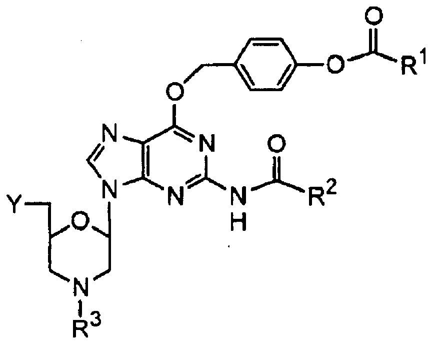 wherein
R¹ is selected from the group consisting of lower alkyl, di(lower alkyl)amino, and phenyl;
R² is selected from the group consisting of lower alkyl, monocyclic arylmethyl, and monocyclic (aryloxy)methyl;
R³ is selected from the group consisting of triarylmethyl and hydrogen; and
Y is a chlorophosphoramidate group or a solid support;
wherein the term lower alkyl defines an alkyl radical of one to six carbon atoms,
provided that the doubly protected guanine morpholino compound does not have the following structure: 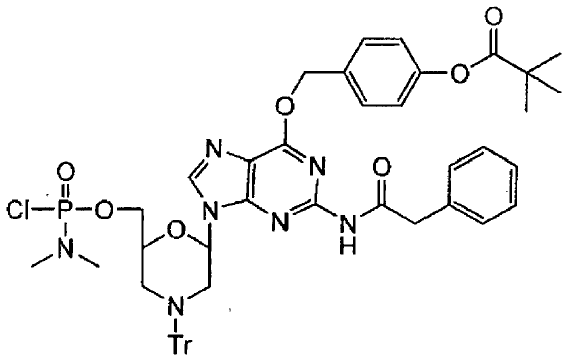 or 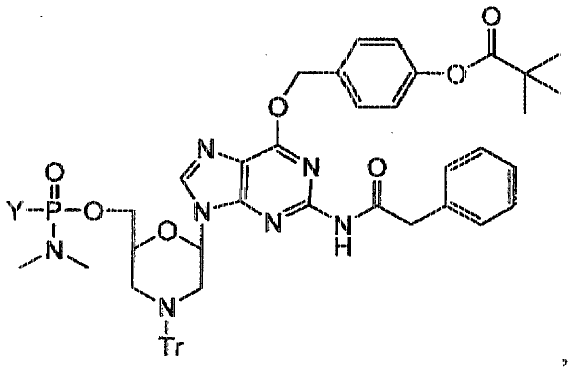
wherein Y is a solid support and Tr is trityl.

10. The method of claim 9, wherein Y is a chlorophosphoramidate group of the form -O-P(=O)-N(CH₃)₂Cl, or wherein R³ is selected from trityl (triphenylmethyl), 4-methoxy-trityl, 4-methyltrityl, 4,4'-dimethyltrityl, and 4,4',4"-trimethyltrityl, or wherein R¹ is lower alkyl or wherein R² is benzyl or -CH(CH₃)₂, wherein the term lower alkyl defines an alkyl radical of one to six carbon atoms.

11. The method of claim 9, wherein R¹ is -C(CH₃)₃.

12. The method of claim 10 or 11, wherein said deprotecting of step (b) comprises exposing said triarylmethyl-protected ring nitrogen to a reagent solution comprising a heterocyclic amine salt in a trifluoroethanol-containing solvent, said salt being a salt of a heterocyclic amine, having a pKa in the range of 1-4 in its protonated form, with an acid selected from a sulfonic acid, trifluoroacetic acid, and hydrochloric acid.

13. A method of synthesizing a morpholino oligomer, the method comprising:
(a) reacting a solid-phase-supported morpholino subunit, having an unprotected ring nitrogen, with a first morpholino subunit monomer, having a triarylmethyl-protected ring nitrogen and an activated phosphoramidate group on a 5'-exocyclic carbon, thereby forming a phosphorodiamidate linkage between said 5'-exocyclic carbon and said unprotected ring nitrogen;
(b) deprotecting said protected ring nitrogen, to form a product comprising an unprotected ring nitrogen;
(c) optionally reacting the product from step (b) with a further morpholino subunit monomer comprising a triarylmethyl-protected morpholino ring nitrogen and an activated phosphoramidate group on a 5'-exocyclic carbon, thereby forming a phosphoramidate linkage between the 5'-exocyclic carbon of the further morpholino subunit monomer and the unprotected morpholino ring nitrogen of the product from step (b);
(d) repeating steps (b) and (c) one or more times with further base-protected morpholino subunit monomers;
wherein said deprotecting comprises exposing said triarylmethyl-protected ring nitrogen to a reagent solution comprising a heterocyclic amine salt in a trifluoroethanol-containing solvent, said salt being a salt of a heterocyclic amine, having a pKa in the range of 1-4 in its protonated form, with an acid selected from a sulfonic acid, trifluoroacetic acid, and hydrochloric acid, and
wherein the heterocyclic amine salt is not 4-cyanopyridinium trifuoroacetate (CYTFA).

14. The method of claim 13, wherein said heterocyclic amine is selected from the group consisting of an electron withdrawing group-substituted pyridine, thiazole, pyridazine, pyrazole, triazole.

15. The method of claim 14, wherein said heterocyclic amine is an electron withdrawing group-substituted pyridine, wherein said heterocyclic amine is a chloro- or cyano-substituted pyridine, or wherein said electron withdrawing group is selected from the group consisting of halogen, cyano, aldehyde, keto, carboxyester, and carboxamide.

16. The method of claim 13, wherein said salt is a salt of a sulfonic acid, selected from an alkylsulfonate, a (fluoroalkyl)sulfonate, and a p-toluenesulfonate, or a trifluoroacetate.

17. The method of claim 12 or 15, wherein said salt is 3-chloropyridinium methanesulfonate (CPM).

18. The method of claim 12 or 15, wherein said solvent comprises dichloromethane and trifluoroethanol in volume ratio in the range of 90:10 to 25:75.

19. The method of claim 18, wherein said volume ratio is 80:20.

20. The method of claim 13, wherein said triarylmethyl is selected from the group consisting of trityl, 4-methoxytrityl, 4-methyltrityl, 4,4'-dimethyltrityl, and 4,4',4"-trimethyltrityl.

## Patentansprüche

1. Morpholino-Verbindung mit der folgenden Struktur 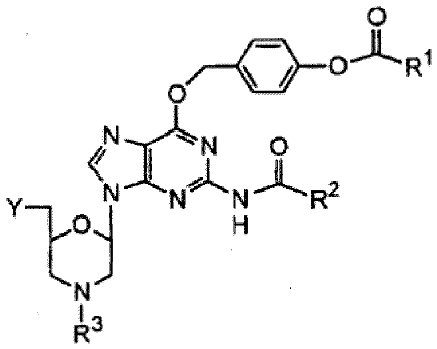 wobei:
R¹ ausgewählt ist aus der Gruppe bestehend aus Niederalkyl, Di(niederalkyl)amino, und Phenyl;
R² ausgewählt ist aus der Gruppe bestehend aus Niederalkyl, monozyklisches Arylmethyl, und monozyklisches (Aryloxy)methyl;
R³ ausgewählt ist aus der Gruppe bestehend aus Triarylmethyl und Wasserstoff; und
Y ausgewählt ist aus der Gruppe bestehend aus: eine geschützte oder ungeschützte Hydroxyl- oder Amino-Gruppe; eine Chlorphosphoramidat-Gruppe; eine Phosphordiamidat-Verknüpfung mit dem Morpholino-Ring-Stickstoff einer weiteren Morpholino-Verbindung, eine Phosphordiamidat-Verknüpfung mit dem Morpholino-Ring-Stickstoff einer Morpholino-Untereinheit in einem Morpholino-Oligomer oder ein fester Träger;
wobei der Begriff Niederalkyl einen Alkylrest mit einem bis sechs Kohlenstoffatom(en) definiert;
vorausgesetzt, dass die Verbindung nicht die folgende Struktur hat: 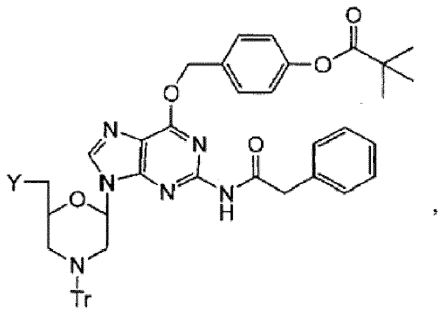 wobei:
Y -OH, 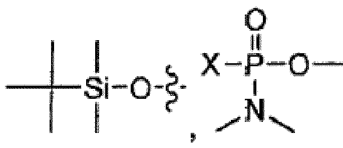 ist, wobei X Cl, der Morpholino-Ring-Stickstoff einer weiteren Morpholino-Verbindung, der Morpholino-Ring-Stickstoff einer Morpholino-Untereinheit in einem Morpholino-Oligomer oder ein fester Träger ist; und
TR Trityl ist.

2. Verbindung nach Anspruch 1, wobei Y ausgewählt ist aus der Gruppe bestehend aus einer geschützten oder ungeschützten Hydroxyl-Gruppe und einer Chlorphosphoramidat-Gruppe.

3. Verbindung nach Anspruch 1, wobei Y eine Trialkylsilyl-geschützte Hydroxylgruppe ist.

4. Verbindung nach Anspruch 1, wobei Y eine Chlorphosphoramidat-Gruppe der Formel -O-P(= O)-N(CH₃)₂Cl ist.

5. Verbindung nach Anspruch 1, wobei R³ ausgewählt ist aus Trityl, 4-Methoxytrityl, 4-Methyltrityl, 4,4'-Dimethyltrityl, und 4,4',4"-Trimethyltrityl.

6. Verbindung nach Anspruch 1, wobei R¹ ein Niederalkyl ist, wobei der Begriff Niederalkyl einen Alkylrest mit einem bis sechs Kohlenstoffatomen definiert.

7. Verbindung nach Anspruch 6, wobei R¹-C(CH₃)₃ ist.

8. Verbindung nach Anspruch 1, wobei R² Benzyl oder -CH(CH₃)₂ ist.

9. Verfahren zur Synthese eines Morpholino-Oligomers, das Verfahren umfassend:
(a) Umsetzen einer Festphasen-unterstützten Morpholino-Untereinheit, welche einen ungeschützten Ring-Stickstoff hat, mit einem ersten Morpholino-Untereinheits-Monomer, welches einen Triarylmethyl-geschützten Ring-Stickstoff und eine aktivierte Phosphoramidat-Gruppe an einem 5'-exozyklischen Kohlenstoff hat, wodurch eine Phosphordiamidat-Verknüpfung zwischen dem 5'-exozyklischen Kohlenstoff und dem ungeschützten Ring-Stickstoff gebildet wird;
(b) Entschützen des geschützten Ring-Stickstoffs, um ein Produkt zu bilden, welches einen ungeschützten Ring-Stickstoff umfasst;
(c) gegebenenfalls Umsetzen des Produkts aus Schritt (b) mit einem weiteren Morpholino-Untereinheits-Monomer, welches einen Triarylmethyl-geschützten Morpholino-Ring-Stickstoff und eine aktivierte Phosphoramidat-Gruppe an einem 5'- exozyklischen Kohlenstoff umfasst, wodurch eine Phosphoramidat-Verknüpfung zwischen dem 5'-exozyklischen Kohlenstoff des weiteren Morpholino-Untereinheits-Monomers und dem ungeschützten Morpholino-Ring-Stickstoff des Produkts aus Schritt (b) gebildet wird; und
(d) gegebenenfalls Wiederholen der Schritte (b) und (c), ein oder mehrere Male; wobei mindestens Einer von dem ersten Morpholino-Untereinheits-Monomer, dem weiteren Morpholino-Untereinheits-Monomer oder dem Festphasen-unterstützten Morpholino-Untereinheits-Monomer eine zweifach geschützte Guanin-Morpholino-Verbindung ist mit der Struktur: 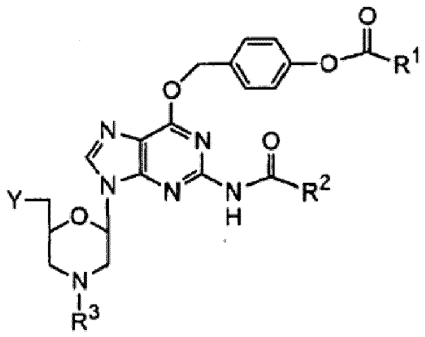 wobei
R¹ ausgewählt ist aus der Gruppe bestehend aus Niederalkyl, Di(niederalkyl)amino, und Phenyl;
R² ausgewählt ist aus der Gruppe bestehend aus Niederalkyl, monozyklisches Arylmethyl, und monozyklisches (Aryloxy)methyl;
R³ ausgewählt ist aus der Gruppe bestehend aus Triarylmethyl und Wasserstoff; und
Y eine Chlorophosphoramidat-Gruppe oder ein fester Träger ist;
wobei der Begriff Niederalkyl einen Alkylrest mit einem bis sechs Kohlenstoffatom(en) definiert,
vorausgesetzt, dass die zweifach geschützte Guanin-Morpholino-Verbindung nicht die folgende Struktur hat: 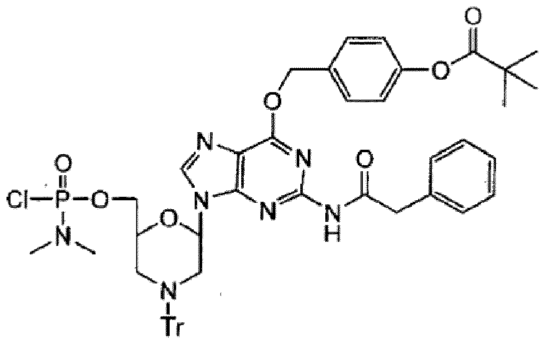 oder 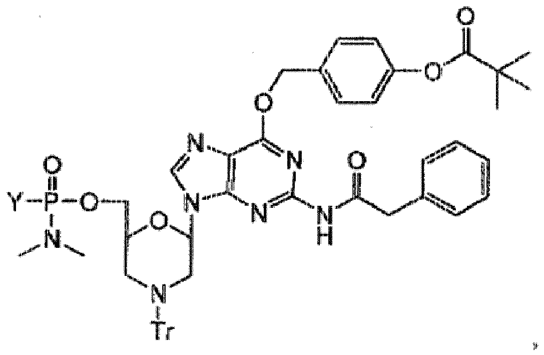
wobei Y ein fester Träger ist und Tr Trityl ist.

10. Verfahren nach Anspruch 9, wobei Y eine Chlorophosphoramidat-Gruppe der Formel -O-P(=O)-N(CH₃)₂Cl ist, oder wobei R³ ausgewählt ist aus Trityl (Triphenylmethyl), 4-Methoxy-Trityl, 4-Methyltrityl, 4,4'-Dimethyltrityl, und 4,4',4"-Trimethyltrityl, oder worin R¹ Niederalkyl ist oder worin R² Benzyl oder -CH(CH₃)₂ ist, wobei der Begriff Niederalkyl einen Alkylrest mit einem bis sechs Kohlenstoffatom(en) definiert.

11. Verfahren nach Anspruch 9, wobei R¹-C(CH₃)₃ ist.

12. Verfahren nach Anspruch 10 oder 11, wobei das Entschützen in Schritt (b) umfasst den Triarylmethyl-geschützten Ring-Stickstoffs einer Reagenz-Lösung auszusetzen, welche ein heterozyklisches Aminsalz in einem Trifluorethanolhaltigen Lösungsmittel umfasst, wobei das Salz ein Salz ist eines heterozyklischen Amins, mit einem pKs im Bereich von 1-4 in seiner protonierten Form, mit einer Säure, ausgewählt aus Sulfonsäure, Trifluoressigsäure, und Salzsäure.

13. Verfahren zur Synthese eines Morpholino-Oligomers, das Verfahren umfassend:
(a) Umsetzen einer Festphasen-unterstützten Morpholino-Untereinheit, welche einen ungeschützten Ring-Stickstoff hat, mit einem ersten Morpholino-Untereinheits-Monomer, welches einen Triarylmethyl-geschützten Ring-Stickstoff und eine aktivierte Phosphoramidat-Gruppe an einem 5'-exozyklischen Kohlenstoff hat, wodurch eine Phosphordiamidat-Verknüpfung zwischen dem 5'-exozyklischen Kohlenstoff und dem ungeschützten Ring-Stickstoff gebildet wird;
(b) Entschützen des geschützten Ring-Stickstoffs, um ein Produkt zu bilden, welches einen ungeschützten Ring-Stickstoff umfasst;
(c) gegebenenfalls Umsetzen des Produkts aus Schritt (b) mit einem weiteren Morpholino-Untereinheits-Monomer, welches einen Triarylmethyl-geschützten Morpholino-Ring-Stickstoff und eine aktivierte Phosphoramidat-Gruppe an einem 5'-exozyklischen Kohlenstoff umfasst, wodurch eine Phosphoramidat-Verknüpfung zwischen dem 5'-exozyklischen Kohlenstoff des weiteren Morpholino-Untereinheits-Monomers und dem ungeschützten Morpholino-Ring-Stickstoff des Produkts aus Schritt (b) gebildet wird; und
(d) Wiederholen der Schritte (b) und (c), ein oder mehrere Male, mit weiteren Basen-geschützten Morpholino-Untereinheits-Monomeren;
wobei das Entschützen umfasst den Triarylmethyl-geschützten Ring-Stickstoffs einer Reagenz-Lösung auszusetzen, welche ein heterozyklisches Aminsalz in einem Trifluorethanol-haltigen Lösungsmittel umfasst, wobei das Salz ein Salz ist eines heterozyklischen Amins, mit einem pKs im Bereich von 1-4 in seiner protonierten Form, mit einer Säure, ausgewählt aus Sulfonsäure, Trifluoressigsäure, und Salzsäure, und
wobei das heterozyklische Aminsalz nicht 4-Cyanopyridiniumtrifuoroacetat (CYTFA) ist.

14. Verfahren nach Anspruch 13, wobei das heterozyklische Amin ausgewählt ist aus der Gruppe bestehend aus, mit einer Elektronen-abziehenden Gruppe substituiertes, Pyridin, Thiazol, Pyridazin, Pyrazol, Triazol.

15. Verfahren nach Anspruch 14, wobei das heterozyklische Amin ein elektronenziehend-substituiertes Pyridin ist, wobei das heterozyklische Amin ein Chlor- oder Cyano-substituiertes Pyridin ist, oder wobei die Elektronen-ziehende Gruppe ausgewählt ist aus der Gruppe bestehend aus Halogen, Cyano, Aldehyd, Keto, Carboxyester, und Carboxamid.

16. Verfahren nach Anspruch 13, wobei das Salz ein Salz einer Sulfonsäure ist, ausgewählt aus einem Alkylsulfonat, einem (Fluoralkyl)sulfonat, und einem p-Toluolsulfonat, oder einem Trifluoracetat.

17. Verfahren nach Anspruch 12 oder 15, wobei das Salz 3-Chlorpyridinium-Methansulfonat (CPM) ist.

18. Verfahren nach Anspruch 12 oder 15, wobei das Lösungsmittel Dichlormethan und Trifluorethanol in einem Volumenverhältnis im Bereich von 90:10 bis 25:75 umfasst.

19. Verfahren nach Anspruch 18, wobei das Volumenverhältnis 80:20 beträgt.

20. Verfahren nach Anspruch 13, wobei das Triarylmethyl ausgewählt ist aus der Gruppe bestehend aus Trityl, 4-Methoxytrityl, 4-Methyltrityl, 4,4'-Dimethyltrityl und 4,4',4"-Trimethyltrityl.

## Revendications

1. Composé morpholino ayant la structure suivante 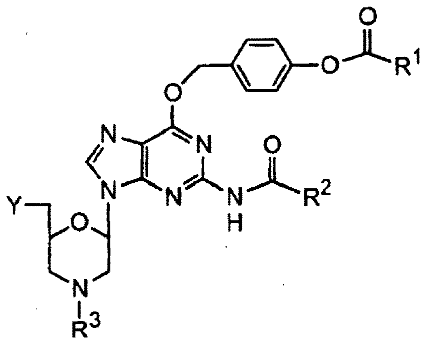 dans laquelle :
R¹ est sélectionné parmi le groupe constitué d'alkyle inférieur, de diaminoalkyle inférieur, et de phényle ;
R² est sélectionné parmi le groupe constitué d'alkyle inférieur, d'arylméthyle monocyclique, et
d'(aryloxy)méthyle monocyclique ;
R³ est sélectionné parmi le groupe constitué de triarylméthyle et d'hydrogène ; et
Y est sélectionné parmi le groupe constitué de : un groupe hydroxyle ou amino protégé ou non protégé ; un groupe chlorophosphoramidate ; une liaison phosphorodiamidate à l'azote de cycle morpholino d'un composé morpholino supplémentaire, une liaison phosphorodiamidate à l'azote de cycle morpholino d'une sous-unité morpholino dans un oligomère morpholino ou un support solide ;
dans laquelle le terme alkyle inférieur définit un radical alkyle d'un à six atomes de carbone ;
à la condition que le composé n'ait pas la structure suivante : 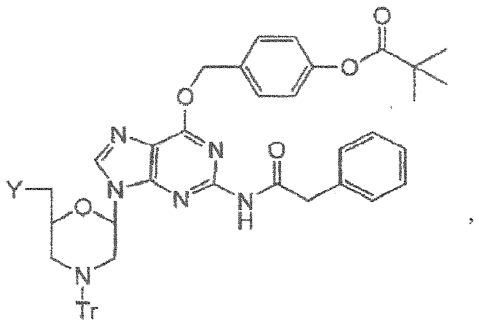
dans laquelle,
Y est -OH, 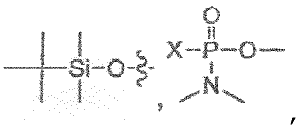 dans laquelle X est Cl, l'azote de cycle morpholino d'un composé morpholino supplémentaire, l'azote de cycle morpholino d'une sous-unité morpholino dans un oligomère morpholino ou un support solide ; et
Tr est trityle.

2. Composé selon la revendication 1, dans lequel Y est sélectionné parmi le groupe constitué d'un groupe hydroxyle protégé ou non protégé et d'un groupe chlorophosphoramidate.

3. Composé selon la revendication 1, dans lequel Y est un groupe hydroxyle protégé par trialkylsilyle.

4. Composé selon la revendication 1, dans lequel Y est un groupe chlorophosphoramidate de la forme -O-P(=O)-N(CH₃)₂Cl.

5. Composé selon la revendication 1, dans lequel R³ est sélectionné parmi trityle, 4-méthoxytrityle, 4-méthyltrityle, 4,4'-diméthyltrityle, et 4,4',4"-triméthyltrityle.

6. Composé selon la revendication 1, dans lequel R¹ est un alkyle inférieur, dans lequel le terme alkyle inférieur définit un radical alkyle d'un à six atomes de carbone.

7. Composé selon la revendication 6, dans lequel R¹ est - C(CH₃)₃.

8. Composé selon la revendication 1, dans lequel R² est benzyle ou -CH(CH₃)₂.

9. Procédé de synthèse d'un oligomère morpholino, le procédé comprenant les étapes consistant à :
(a) mettre à réagir une sous-unité morpholino supportée en phase solide, ayant un azote de cycle non protégé, avec un premier monomère d'une sous-unité morpholino ayant un azote de cycle protégé par triarylméthyle et un groupe phosphoramidate activé sur un carbone exocyclique en 5', formant ainsi une liaison phosphorodiamidate entre ledit carbone exocyclique en 5' et ledit azote de cycle non protégé ;
(b) déprotéger ledit azote de cycle protégé, pour former un produit comprenant un azote de cycle non protégé ;
(c) éventuellement mettre à réagir le produit de l'étape (b) avec un monomère d'une sous-unité morpholino supplémentaire comprenant un azote de cycle morpholino protégé par triarylméthyle et un groupe phosphoramidate activé sur un carbone exocyclique en 5', formant ainsi une liaison phosphoramidate entre le carbone exocyclique en 5' du monomère d'une sous-unité morpholino supplémentaire et l'azote de cycle morpholino non protégé du produit de l'étape (b) ; et
(d) éventuellement répéter les étapes (b) et (c) une ou plusieurs fois ;
dans lequel au moins un du premier monomère d'une sous-unité morpholino, du monomère d'une sous-unité morpholino supplémentaire ou du monomère d'une sous-unité morpholino supportée en phase solide est un composé morpholino guanine doublement protégé ayant la structure : 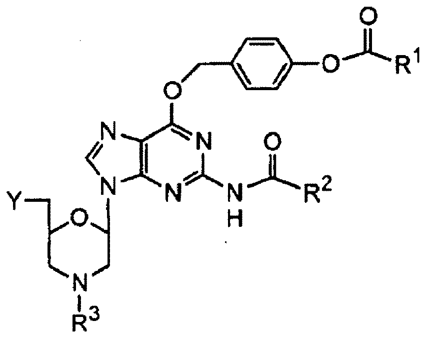 dans laquelle :
R¹ est sélectionné parmi le groupe constitué d'alkyle inférieur, de diaminoalkyle inférieur, et de phényle ;
R² est sélectionné parmi le groupe constitué d'alkyle inférieur, d'arylméthyle monocyclique, et d'(aryloxy)méthyle monocyclique ;
R³ est sélectionné parmi le groupe constitué de triarylméthyle et d'hydrogène ; et
Y est un groupe chlorophosphoramidate ou un support solide ;
dans laquelle le terme alkyle inférieur définit un radical alkyle d'un à six atomes de carbone,
à la condition que le composé morpholino guanine doublement protégé n'ait pas la structure suivante : 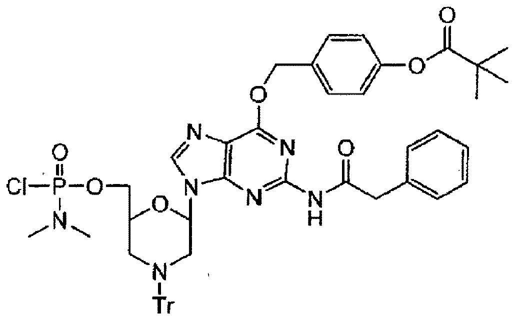 ou 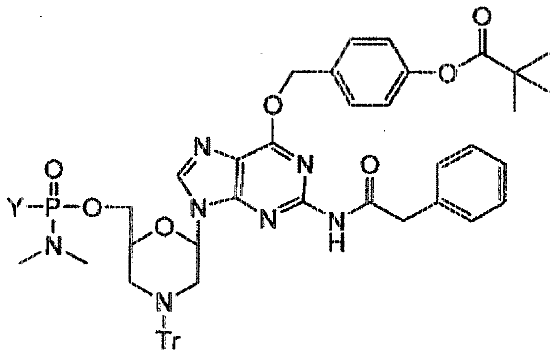 dans lesquelles Y est un support solide et Tr est trityle.

10. Procédé selon la revendication 9, dans lequel Y est un groupe chlorophosphoramidate de la forme -O-P(=O)-N(CH₃)₂Cl, ou dans lequel R³ est sélectionné parmi trityl(triphénylméthyle), 4-méthoxytrityle, 4-méthyltrityle, 4,4'-diméthyltrityle, et 4,4',4"-triméthyltrityle, ou dans lequel R¹ est alkyle inférieur ou dans lequel R² est benzyle ou -CH(CH₃)₂, dans lequel le terme alkyle inférieur définit un radical alkyle d'un à six atomes de carbone.

11. Procédé selon la revendication 9, dans lequel R¹ est - C(CH₃)₃.

12. Procédé selon la revendication 10 ou la revendication 11, dans lequel ladite déprotection de l'étape (b) comprend l'étape consistant à exposer ledit azote de cycle protégé par triarylméthyle à une solution de réactif comprenant un sel d'amine hétérocyclique dans un solvant contenant du trifluoroéthanol, ledit sel étant un sel d'une amine hétérocyclique, ayant une pKa dans la gamme de 1 - 4 dans sa forme protonée, avec un acide sélectionné parmi un acide sulfonique, un acide trifluoroacétique, et un acide chlorhydrique.

13. Procédé de synthèse d'un oligomère morpholino, le procédé comprenant les étapes consistant à :
(a) mettre à réagir une sous-unité morpholino supportée en phase solide, ayant un azote de cycle non protégé, avec un premier monomère d'une sous-unité morpholino, ayant un azote de cycle protégé par triarylméthyle et un groupe phosphoramidate activé sur un carbone exocyclique en 5', formant ainsi une liaison phosphorodiamidate entre ledit carbone exocyclique en 5' et ledit azote de cycle non protégé ;
(b) déprotéger ledit azote de cycle protégé, pour former un produit comprenant un azote de cycle non protégé ;
(c) éventuellement mettre à réagir le produit de l'étape (b) avec un monomère d'une sous-unité morpholino supplémentaire comprenant un azote de cycle morpholino protégé par triarylméthyle et un groupe phosphoramidate activé sur un carbone exocyclique en 5', formant ainsi une liaison phosphoramidate entre le carbone exocyclique en 5' du monomère d'une sous-unité morpholino supplémentaire et l'azote de cycle morpholino non protégé du produit de l'étape (b) ;
(d) répéter les étapes (b) et (c) une ou plusieurs fois avec des monomères d'une sous-unité morpholino protégée par une base supplémentaires ;
dans lequel ladite déprotection comprend l'étape consistant à exposer ledit azote de cycle protégé par triarylméthyle à une solution de réactif comprenant un sel d'amine hétérocyclique dans un solvant contenant du trifluoroéthanol, ledit sel étant un sel d'une amine hétérocyclique, ayant une pKa dans la gamme de 1 - 4 dans sa forme protonée, avec un acide sélectionné parmi un acide sulfonique, un acide trifluoroacétique, et un acide chlorhydrique, et
dans lequel le sel d'amine hétérocyclique n'est pas du 4-cyanopyridinium trifluoroacétate (CYTFA).

14. Procédé selon la revendication 13, dans lequel ladite amine hétérocyclique est sélectionnée parmi le groupe constitué d'une pyridine substituée par un groupe de retrait d'électrons, d'un thiazole, d'une pyridazine, d'un pyrazole, d'un triazole.

15. Procédé selon la revendication 14, dans lequel ladite amine hétérocyclique est une pyridine substituée par un groupe de retrait d'électrons, dans lequel ladite amine hétérocyclique est une pyridine chloro- ou cyano-substituée, ou dans lequel ledit groupe de retrait d'électrons est sélectionné parmi le groupe constitué d'halogène, de cyano, d'aldéhyde, de cétone, de carboxyester, et de carboxamide.

16. Procédé selon la revendication 13, dans lequel ledit sel est un sel d'un acide sulfonique, sélectionné parmi un alkylsulfonate, un (fluoroalkyl)sulfonate, et un p-toluènesulfonate, ou un trifluoroacétate.

17. Procédé selon la revendication 12 ou la revendication 15, dans lequel ledit sel est du 3-chloropyridinium méthanesulfonate (CPM).

18. Procédé selon la revendication 12 ou la revendication 15, dans lequel ledit solvant comprend du dichlorométhane et du trifluoroéthanol dans un rapport volumique dans la gamme de 90 : 10 à 25 : 75.

19. Procédé selon la revendication 18, dans lequel ledit rapport volumique est 80 : 20.

20. Procédé selon la revendication 13, dans lequel ledit triarylméthyle est sélectionné parmi le groupe constitué de trityle, 4-méthoxytrityle, 4-méthyltrityle, 4,4'-diméthyltrityle, et 4,4',4"-triméthyltrityle.
